# EUROPEAN PATENT APPLICATION

(11) **EP 1 920 780 A1**
(43) Date of publication of application: **14.05.2008**
(21) Application number: 06021470.7
(22) Date of filing: 12.10.2006
(51) Int. Cl.: A61K 38/04, A61P 21/00, A61P 19/00, A61P 25/14

(54) **Peptide compounds for the treatment of hyperexcitability disorders**

(71) Applicant: SCHWARZ PHARMA AG, 40789 Monheim (DE)
(72) Inventor: Heers, Cara, 64293 Darmstadt (DE); Stöhr, Thomas, 40789 Monheim (DE); Beyreuther, Bettina, 40219 Düsseldorf (DE)
(74) Representative: Weiss, Wolfgang

(57) **Abstract**

The present invention is directed to the use of a class of peptide compounds for treating diseases associated with hyperexcitability, preferably a disease associated with a hyperexcitable tissue. The disease might be associated with dysfunction of ion channels or/and the disease might be an anxiety or/and stress disease.

## Description

The present invention is directed to the use of a class of peptide compounds for treating diseases associated with hyperexcitability, preferably diseases associated with a hyperexcitable tissue.

Certain peptides are known to exhibit central nervous system (CNS) activity and are useful in the treatment of epilepsy and other CNS disorders. These peptides are described in the U.S. Patent No. 5,378,729 and in U.S. Patent No. 5,773,475, which are hereby incorporated by reference.

WO 02/074297 relates to the use of peptidic compounds for the preparation of pharmaceutical compositions useful for the treatment of allodynia related to peripheral neuropathic pain. WO 02/074784 relates to the use of peptidic compounds showing antinociceptive properties for treating different types and symptoms of acute and chronic pain, especially non neuropathic inflammatory pain, e.g. rheumatoid arthritic pain or/and secondary inflammatory osteo-arthritic pain.

Hyperexcitability is defined herein as an abnormal increase in responsiveness of a central or peripheral nervous system neuron to synaptic input. In addition, hyperexcitability is also referred to as an abnormal increase in responsiveness of any excitable membrane, such as a muscle cell membrane, to a physiological signal or to excitotoxicity caused by a pathophysiological signal.

Examples for hyperexcitable tissues are all innervated tissues such as central or peripheral nervous tissue, muscle tissue, and other organ tissue.

Examples for diseases associated with hyperexcitability are channelopathies, anxiety- and stress-diseases.

Hyperexcitability can be induced by dysfunction of ion channels. According to their mode of regulation, ion channels can be divided into voltage-gated ion channels and ligand-gated ion channels. Ligand-gated ion channels are also referred to as receptors. Examples for voltage-gated ion channels are voltage-gated sodium channels, voltage-gated calcium channels, voltage-gated potassium channels, and voltage-gated chloride channels. Examples for ligand-gated ion channels are nicotinic acetylcholine receptors, ryanodine receptors (calcium release channels), cyclic nucleotide-gated receptors, ATP-receptors, GABA-A receptors, glutamate-NMDA receptors, glycine-receptors, 5-HT3-receptors, and pH sensitive channels such as acid-sensing ion channel (ASIC), and TRP receptors.

Ion channel dysfunction may have genetic or other causes, such as tissue damage.

Diseases caused by one or more mutations of genes coding for ion channel subunits or proteins that regulate them are referred to as channelopathies. There are a large number of distinct dysfunctions known to be caused by ion channel mutations. They comprise a heterogenous group of usually hereditary disorders which in most cases are clinically characterized by episodes of disturbed excitability of nerve or muscle cells. The genes for the construction of ion channels are highly conserved amongst mammals and one condition, hyperkalemic periodic paralysis, was first identified in the descendants of Impressive, a pedigree race horse. Well known examples of identified channelopathies are diseases of the skeletal muscle (such as hyper-, hypo- and normokalemic (high, low and normal potassium blood concentrations) periodic paralysis, paroxysmal dystonia, myotonia congenita and paramyotonia congenita), central nervous disorders of excitability (such as episodic ataxias and several forms of inherited epilepsies), and cardiac arrhythmias (such as long QT syndromes).

Ion channels dysfunctions can also be caused by variations in ion channel genes that are not sufficiently severe to be classified as mutations, but instead are referred to as polymorphisms. Such polymorphisms may contribute to unique drug responses in carriers of these gene variants (Kass, RS, J Clin Invest (2005), 115:1986-1989).

Voltage-gated sodium channels are responsible for the generation and propagation of action potentials in excitable cells. The excitability of tissues depends mainly on the number of voltage-gated sodium channels that are available for activation. The fraction of sodium channels available for activation is regulated by fast inactivation, which occurs on a millisecond time scale, and slow inactivation occurring within seconds or minutes.

Mutations in genes coding for sodium channels are known to cause a number of characteristic diseases. Most inherited sodium channel mutations that are associated with human disease alter the inactivation process and hence alter the essential control of electrical-impulse duration that is effected by transition into the inactivated state (Jurkat-Rott, K, J Clin Invest (2005), 115:2000-2009). Most well characterised mutations are at the SCN4A sodium channel gene which codes for the alpha-subunit of the skeletal muscle sodium channel. Following diseases are listed in the OMIM database of NCBI for the SCN4A gene:
- Cramps, familial, potassium-aggravated MIM: 603967
- Hyperkalemic periodic paralysis MIM: 170500
- Hypokalemic periodic paralysis MIM: 170400
- Myotonia congenita, atypical, acetazolamide-responsive MIM: 608390
- Paramyotonia congenita MIM: 168300.

Dystonia (literally, "abnormal muscle tone") is a generic term used to describe a neurological movement disorder involving involuntary, sustained muscle contractions. Dystonia may affect muscles throughout the body (generalised), in certain parts of the body (segmental), or may be confined to particular muscles or muscle groups (focal). Primary dystonia is caused by a pathology of the central nervous system, likely originating in those parts of the brain concerned with motor function, such as the basal ganglia. An example for dystonia associated with dysfunction of the voltage-gated sodium channel is paroxysmal dystonia.

Muscle weakness (or "lack of strength") is the inability to exert force with ones muscles to the degree that would be expected given the individual's general physical fitness. A test of strength is often used during a diagnosis of a muscular disorder before the etiology can be identified.

The term subsumes two other more specific terms, *true weakness* and *perceived weakness.* True weakness (or "objective weakness") describes a condition where the instantaneous force exerted by the muscles is less than would be expected. For instance, if a patient suffers from amyotrophic lateral sclerosis (ALS), motor neurons are damaged and can no longer stimulate the muscles to exert normal force. Perceived weakness (or "subjective weakness") describes a condition where it seems to the patient that more effort than normal is required to exert a given amount of force. For instance, a person with chronic fatigue syndrome may struggle to climb a set of stairs when feeling especially fatigued, but if their muscle strength is objectively measured (eg, the maximum weight they can press with their legs) it is essentially normal. In some conditions such as myasthenia gravis muscle strength is normal when resting, but *true weakness* occurs after the muscle has been subjected to exercise.

Myotonia is a neuromuscular disorder characterized by the slow relaxation of the muscles after voluntary contraction or electrical stimulation. Generally, repeated effort is needed to relax the muscles, and the condition improves after the muscles have warmed-up. However, prolonged, rigorous exercise may also trigger the condition. Individuals with the disorder may have trouble releasing their grip on objects or may have difficulty rising from a sitting position and a stiff, awkward gait. During pregnancy, symptoms of myotonia are more frequently experienced in women.

Myotonia can affect all muscle groups. It may be acquired or inherited, and is caused by an abnormality in the muscle membrane. Myotonia is a symptom commonly seen in patients with myotonic muscular dystrophy and channelopathies. Myotonia arising from channelopathies can be exacerbated by exposure to cold, by eating foods that are potassium-rich (such as bananas), and with exertion.

Myasthenias are a group of disorders which exhibit several striking features the essential one being a fluctuating weakness and fatigability of muscle. There is usually some degree of weakness at all times but it is made worse by activity. The weakness and fatigability reflect physiologic abnormalities of the neuromuscular junction that are demonstrated by clinical signs and special electrophysiologic testing.

Paralysis is the complete loss of muscle function for one or more muscle groups. Major causes are stroke, trauma, poliomyelitis, amyotrophic lateral sclerosis (ALS), botulism, spina bifida, and Guillain-Barré syndrome. Paralysis is most often caused by damage to the nervous system or brain, especially the spinal cord. Paralysis often includes loss of feeling in the affected area.

Paramyotonia Congenita (PC) is a rare congenital autosomal dominant neuromuscular disorder characterized by "paradoxical" myotonia. This type of myotonia has been termed paradoxical because it becomes worse with exercise whereas classical myotonia, as seen in myotonia congenita, is alleviated by exercise. PC is also distinguished as it can be induced by cold temperatures. Although more typical of the periodic paralytic disorders, patients with PC may also have potassium provoked paralysis. PC typically presents within the first decade of life and has 100% penetrance. Patients with this disorder commonly present with myotonia in the face or upper extremities. The lower extremities are generally less affected. While some other related disorders result in muscle atrophy, this is not normally the case with PC. This disease can also present as hyperkalemic periodic paralysis and there is debate as to whether the two disorders are actually distinct. Patients typically complain of muscle stiffness that can continue to focal weakness. This muscle stiffness cannot be walked-off, in contrast to myotonia congenita. These symptoms are increased (and sometimes induced) in cold environments. For example, some patients have reported that eating ice cream leads to a stiffening of the throat. For other patients, exercise consistently induces symptoms of myotonia and/or weakness. Typical presentations of this are during squating or repetitive fist clenching. Some patients also indicate that specific foods are able to induce symptoms of paramyotonia congenita. Isolated cases have reported that carrots and watermelon are able to induce these symptoms. The canonical definition of this disorder precludes permenant weakness in the definition of this disorder. In practice, however, this has not been strictly adhered to in the literature. Diagnosis of paramyotonia congenita is made upon evaluation of patient symptoms and case history. Myotonia must increase with exercise/movement and usually must worsen in cold temperatures. Patients that present with permanent weakness are normally not characterized as having PC. Electromyography may be used to distinguish between paramyotonia congenita and myotonia congenita. Clinicians may also attempt to provoke episodes or myotonia and weakness/paralysis in patients in order to determine whether the patient has PC, hyperkalemic periodic paralysis, or one of the potassium-aggravated myotonias. Genomic sequencing of the SCN4A gene is the definitive diagnostic determinant.

Some patients do not require treatment to manage the symptoms of paramyotonia congenita. Others, however, require treatment for their muscle stiffness and often find mexiletine to be helpful. Others have found acetazolamide to be helpful as well. Avoidance of myotonia triggering events is also an effective method of mytonia prevention.

Paramyotonia congenita (as well as hyperkalemic periodic paralysis and the potassium-aggravated myotonias) is caused by mutations in SCN4A. The phenotype of patients with these mutations is indicated in Table 1 below. These mutations affect fast inactivation of the encoded sodium channel. There-are also indications that some mutations lead to altered activation and deactivation. The result of these alterations in channel kinetics is that there is prolonged inward (depolarizing) current following muscle excitation. There is also the introduction of a "window current" due to changes in the voltage sensitivity of the channel's kinetics.

**Table 1: Mutations of SCN4A (adapted from Vicart et al., 2005).**

| **Mutation** | **Region** |
|---|---|
| I693T | D2S4-S5 |
| T704M* | D2S5 |
| S804F** | D2S6 |
| A1152D | D3S4-S5 |
| A1156T* | D3S4-S5 |
| V1293I | D3S4 |
| G1306V** | D3-4 |
| T1313A | D3-4 |
| T1313M | D3-4 |
| M1360V* | D4S1 |
| M1370V* | D4S1 |
| L1433R | D4S3 |
| R1448C | D4S4 |
| R1448H | D4S4 |
| | |
| R1448P | D4S4 |
| R1448S | D4S4 |
| R1456E | D4S4 |
| V1458F | D4S4 |
| F1473S | D4S4-S5 |
| M1592V* | D4S6 |
| G1702K | C-term |
| F1795I | C-term |

| | |
|---|---|
| Mutation region nomenclature is: domain number (e.g., D1) followed by segment number (e.g., S4). Thus, D2S3 indicates that the mutation is in the 3^{rd} membrane spanning loop of the 2^{nd} domain. Some mutations occur between segments and are denoted similarly (e.g., D4S4-S5 occurs between the 4^{th} and 5^{th} segments of the 4^{th} domain). Other mutations are located between domains and are denoted DX-Y where X and Y are domain numbers. C-term refers to the carboxy-terminus. * Symptoms of both PC and hyperKPP (Periodica paralytica paramyotonica) ** Also diagnosed as a Potassium-aggravated myotonia | |

Diseases associated with hyperexcitability might be caused by genetic dysfunction of voltage- or ligand-gated ion channels, such as voltage-gated calcium channels, voltage-gated potassium channels, voltage-gated chloride channels, nicotinic acetylcholine receptors, ryanodine receptors (calcium release channels), cyclic nucleotide-gated receptors, ATP-receptors, GABA-A receptors, glutamate-NMDA receptors, glycine- receptors, 5-HT3-receptors, and pH sensitive channels such as acid-sensing ion channel (ASIC), and TRP receptors. Diseases associated with dysfunction of these ion channels include, among others, ataxias, myotonias, myasthenias, long QT syndromes, epilepsy syndromes, and hyperthermia.

Examples for further diseases associated with hyperexcitability that might be caused by other reasons than mutations and polymorphisms in genes coding for ion channel subunits or proteins that regulate them are anxiety and stress. Stress and other anxiogenic stimuli can cause hyperexcitability of amygdala neurones (Rainnie et al. J Neuroscience 2004 24(14):3471-3479). Since the amygdala is a key brain center for emotion acute severe or chronic mild stress can result in persistent changes in emotion as for instance found in patients suffering from post-traumatic stress disorder.

Lacosamide does not exert its effects on neuronal excitability by affecting fast inactivation gating of voltage-gated Na⁺ channels (Errington et al., Neuropharmacology, 2006, 50:1016-1029).

The present invention demonstrates that the compounds of Formulae (I), (II), or/and (III) as defined herein, in particular lacosamide, are capable of selectively enhancing slow inactivation of voltage-gated sodium channels while leaving activation and fast inactivation behaviour normal. This constitutes a novel mechanism of action of the compounds of Formulae (I), (II), or/and (III), which thus can positively influence diseases associated with hyperexcitability. Due to this novel mechanism of action, the compounds of Formulae (I), (II), or/and (III) can efficiently normalize excessive sodium channel function such as a reduced slow inactivation which is e.g. seen in mutated channels. Further, this novel mechanism of action can compensate excessive function of other ion channels such as voltage-gated calcium channels, voltage-gated potassium channels, voltage-gated chloride channels, nicotinic acetylcholine receptors, ryanodine receptors (calcium release channels), cyclic nucleotide-gated receptors, ATP-receptors, GABA-A receptors, glutamate-NMDA receptors, glycine- receptors, 5-HT3-receptors, and pH sensitive channels such as acid-sensing ion channel (ASIC), and TRP receptors.

The mode of action of the compounds of Formulae (I), (II) or/and (III) differs from that of common drugs used for the treatment of voltage-gated sodium channel associated diseases. Common drugs used for the treatment of voltage-gated sodium channel associated diseases often affect fast inactivation of voltage-gated sodium channels and therefore affect signal propagation in excitable tissues. In contrast, the compounds of Formulae (I), (II) or/and (III) cause a shift of the curve of slow inactivation to more negative potentials, which reduces excitability, but does not affect signal propagation.

Lehmann-Horn et al. (Current Neurology and Neuroscience Reports (2002) 2:61-69) report that the common mechanism for inexcitability in all known episodic-weakness phenotypes, such as episodic familial periodic paralysis, is a long-lasting depolarization that inactivates sodium ion channels. By the compounds of the present invention, inactivation may be restored at least partially in such patients.

The use of compounds of Formulae (I), (II), or/and (III) for the treatment of a disease associated with hyperexcitability has not been reported. Thus, the present invention concerns the use of the compounds of Formula (I), (II), or/and (III) for the preparation of a pharmaceutical composition for the prevention, alleviation or/and treatment of a disease associated with hyperexcitability.

In a preferred embodiment, the disease associated with hyperexcitability is a disease associated with a hyperexcitable tissue.

Preferably, the disease associated with hyperexcitability is a disease associated with dysfunction of an ion channel, preferably a voltage-gated ion channel or a ligand-gated ion channel.

In a preferred embodiment of the present invention, the disease associated with dysfunction of an ion channel is associated with dysfunction of a voltage-gated ion channel, selected from a voltage-gated sodium channel, a voltage-gated calcium channel, a voltage-gated potassium channel, and a voltage-gated chloride channel or with dysfunction of a ligand-gated ion channel, selected from a nicotinic acetylcholine receptor, a ryanodine receptor, a cyclic nucleotide-gated receptor, an ATP-receptor, a GABA-A receptor, a glutamate-NMDA receptor, a glycine-receptor, a 5-HT3-receptor, and a pH sensitive channel.

More preferably, the disease associated with dysfunction of an ion channel is associated with dysfunction of a voltage-gated sodium channel. Even more preferably, the disease associated with dysfunction of a voltage-gated sodium channel is a disease associated with altered inactivation of voltage-gated sodium channels in comparison to a healthy subject, in particular altered slow inactivation.

Also preferred is that the curve of slow inactivation is affected, in particular the curve of slow inactivation is shifted to depolarized potentials compared with the slow inactivation curve of a healthy subject. Such functional alterations may be reversed completely or at least partially by the compounds of the present invention.

The voltage-gated sodium channel may be any known type of voltage-gated sodium channels, in particular any type expressed in excitable tissues, such as muscle and nerve. Examples of genes encoding voltage-gated sodium channels or/and subunits thereof include SCN1A, (Naᵥ 1.1), SCN2A (Naᵥ 1.2), SCN4A (Naᵥ 1.4), SCN5A (Naᵥ 1.5), SCN8A (Naᵥ 1.6), and SCN9A (Naᵥ 1.7). Preferably the voltage-gated sodium channel comprises a type IV alpha subunit SCN4A.

It is also preferred that the disease associated with dysfunction of an ion channel is associated with an ion channel different from a voltage-gated sodium channel, such as a voltage-gated calcium channel, a voltage-gated potassium channel, a voltage-gated chloride channel, a nicotinic acetylcholine receptor, a ryanodine receptor, a cyclic nucleotide-gated receptor, an ATP-receptor, a GABA-A receptor, a glutamate-NMDA receptor, a glycine-receptor, a 5-HT3-receptor, or a pH sensitive channel.

Dysfunction of an ion channel different from a voltage-gated sodium channel might cause alteration of electrolyte composition in body fluids, in particular in the intracellular fluid or/and in the extracellular fluid, such as in the plasma, which alteration may for instance influence the activation or/and inactivation state of a sodium channel by altering the resting membrane potential. Alteration of electrolyte composition includes alterations of the Na⁺, K⁺, or/and Cl⁻ concentration or pH. Dysfunction of an ion channel different from a voltage-gated sodium channel might also cause alteration of pre-or/and postsynaptic control, and alteration of synaptic transmitter release or/and postsynaptic transmitter action. Dysfunction of an ion channel different from a voltage-gated sodium channel might lead to a disease associated with hyperexcitability that can be prevented, alleviated or/and treated by a modulator of the activity of a voltage-gated sodium channel.

Examples of genes encoding a voltage-gated calcium channel or/and a subunit thereof include CACNA1A, CACNA1S, CACNA1A, CACNB4. Examples of genes encoding a voltage-gated potassium channel or/and a subunit thereof include KCNA1, KCNQ1, KCNQ2, KCNQ3, KCNQ4, KCNE1, KCNE2, hERG. Examples of a gene encoding a chloride channel or/and a subunit thereof include CLCN1, CLCN5, CLCNKB. Examples of genes encoding a ligand-gated ion channel or/and a subunits thereof include CHRNA4 (nicotinic acetylcholine receptor), RYR1 (ryanodine receptor).

In another preferred embodiment of the present invention, the disease of the present invention is a channelopathy. The channelopathy may be associated with a dysfunction of a voltage-gated sodium channel, a voltage-gated calcium channel, a voltage-gated potassium channel, a voltage-gated chloride channel, a nicotinic acetylcholine receptor, a ryanodine receptor, a cyclic nucleotide-gated receptor, an ATP-receptor, a GABA-A receptor, a glutamate-NMDA receptor, a glycine-receptor, a 5-HT3-receptor, or/and a pH sensitive channel, as described herein, which dysfunction may be caused by a mutation. The channelopathy may be selected from familial potassium-aggravated cramps (e.g. MIM: 603967), myasthenic syndrome (e.g. MIM: 603967), hyperkalemic periodic paralysis (e.g. MIM: 170500), hypokalemic periodic paralysis (e.g. MIM: 170400), atypical, acetazolamide-responsive myotonia congenita (e.g. MIM: 608390), and paramyotonia congenita (e.g. MIM: 168300). The channelopathy may also be associated with at least one of the following mutations of the SCN4A polypeptide: I693T, T704M, S804F, A1152D, A1156T, V1293I, G1306V, T1313A, T1313M, M1360V, M1370V, L1433R, R1448C, R1448H, R1448P, R1448S, R1456E, V1458F, F1473S, M1592V, G1702K, and F1795I.

In another preferred embodiment, the disease of the present invention, in particular the disease associated-with dysfunction of an ion channel, is a muscle disorder.

More preferably the disease is a skeletal muscle disorder or/and a movement disorder, in particular a movement disorder.

Even more preferably the skeletal muscle or/and movement disorder is selected from myotonias and paralyses, preferably selected from inherited myotonia and periodic paralyses, more preferably selected from paramyotonia congenita, potassium aggravated myotonia, myotonia fluctuans, myotonia permanens, acetazolamide responsive myotonia, hyperkalemic periodic paralysis, normokalemic paralysis, paroxysmal dystonia, Morvan syndrome and Isaak syndrome.

Paralysis may be associated with loss of feeling in the affected area. Further, paralysis may be associated with stroke, trauma, poliomyelitis, amyotrophic lateral sclerosis (ALS), botulism, spina bifida, Guillain-Barré syndrome, or/and damage of the nervous system or/and brain, especially the spinal cord.

In a further even more preferred embodiment, the skeletal muscle or/and movement disorder is selected from ataxias, in particular selected from episodic ataxia 2, spinocerebellar ataxia 6, and episodic ataxia.

In another even more preferred embodiment, the skeletal muscle or/and movement disorder is selected from myotonias, in particular selected from Thomsen myotonia, Becker myotonia, myotonia congenita, generalized myotonia, myotonia levior.

The myotonia may be associated with slow relaxation of a muscle after voluntary contraction or electrical stimulation. The myotonia may be selected from acquired and inherited myotonia. The myotonia may be caused by an abnormality in the muscle membrane. The myotonia may be associated with myotonic muscular dystrophy or/and a channelopathy (in particular a channelopathy caused by mutations in the chloride, sodium or potassium ion channels in the muscle membrane).

The myotonia may be exacerbated by exposure to cold, by a potassium-rich diet (such as eating bananas), or/and with exertion, e.g. prolonged, rigorous exercise.

In yet another even more preferred embodiment, the skeletal muscle or/and movement disorder is a myasthenia selected from myokymias and hypokalemic periodic paralysis 1.

In another more preferred embodiment, the muscle disorder is a cardiac arrhythmia selected from long QT syndrome 3, Long QT syndrome 5, Jervell- and Lange-Nielsen syndrome, inducible long QT syndrome, long QT syndrome 1, and long QT syndrome 2.

The disease of the present invention is preferably an epilepsy syndrome selected from generalized epilepsy with febrile seizures plus (GEFS+), severe myoclonic epilepsy in infancy (SMEI), benign familial neonatal infantile seizures (BNIFS), intractable childhood epilepsy with generalized tonic-clonic seizures (ICEGTC), infantile spasms (West syndrome), generalized epilepsy associated with CACNB4 dysfunction, benign familial neonatal convulsions 1, benign familial neonatal convulsions 2, and nocturnal frontal lobe epilepsy.

The disease of the present invention is preferably a pain syndrome selected from erythermalgia and familial rectal pain.

The disease of the present invention is preferably selected from dominant deafness, malignant hyperthermia 5, malignant hyperthermia 1, polycystic kidney disease 1, Dent's disease, Bartter syndrome, central core disorder, and-cortical hyperexcitability associated with CACNA1A.

In another preferred embodiment, the disease associated with hyperexcitability is a sodium channelopathy, which preferably is selected from the diseases listed in Table 2a.

In yet another preferred embodiment, the disease associated with hyperexcitability is a potassium channelopathy, which preferably is selected from the diseases associated with a potassium channel listed in Table 2b.

In a further preferred embodiment, the disease associated with hyperexcitability is a calcium channelopathy, which preferably is selected from the diseases associated with a calcium channel listed in Table 2b.

In yet another preferred embodiment, the disease associated with hyperexcitability is a chloride channelopathy, which preferably is selected from the diseases associated with a chloride channel listed in Table 2b.

In a further preferred embodiment, the disease associated with hyperexcitability is preferably selected from the diseases listed in Table 2c.

### Table 2. Target diseases for selective enhancers of slow inactivation of voltage-gated Na⁺ channels in excitable tissues

**Table 2a. Dysfunction of voltage-gated sodium channels (sodium channelopathies)**

| **AFFECTED TISSUE** | | | |
|---|---|---|---|
| **skeletal muscle** | **cardiac muscle** | **CNS** | **PNS** |
| **inherited myotonia and periodic paralyses** | **cardiac arrhythmia** | **epilepsy syndromes** | **pain syndromes** |
| | SCN5A (Naᵥ 1.5) | | SNC9A (Naᵥ 1.7) |
| SCN4A (Naᵥ 1.4) | long QT syndrome 3 | SCN1A, (Naᵥ 1.1), | erythermalgia (= erythromelagia) *(Yang et al., 2004)* familial rectal pain |
| *(cf. Table 54-1 in "Principles of Neurology" & Lehmann-Horn and Jurkat-Rott www.channelopathies.org)* | | SCN2A (Naᵥ 1.2) generalized | |
| | *(Lehmann-Hom and Jurkat-Rott www. channelopathies.org)* | epilepsy with febrile seizures plus | |
| Paramyotonia congenita | | (GEFS+) | *(Meisler and Kearney, 2005)* |
| Potassium aggravated Potassium aggravated myotonia | | severe myoclonic epilepsy in infancy (SMEI) | |
| Myotonia fluctuans | | benign familial neonatal infantile seizures (BNIFS) | |
| Myotonia permanens | | | |
| Acetazolamide responsive myotonia | | | |
| | | intractable childhood epilepsy with generalized tonic-clonic seizures (ICEGTC) | |
| Hyperkalemic periodic Paralysis | | | |
| Normokalemic paralysis | | | |
| | | infantile spasms (West syndrome) | |
| **movement disorders:** | | | |
| SCN8A (Naᵥ 1.6) | | *(Meisler and Kearney, 2005)* | |
| paroxysmal dystonia | | | |
| *(Siep et al., 2002)* | | | |
| Morvan syndrome | | | |
| *Isaak syndrome* | | | |

**Table 2b. Dysfunction of other voltage-gated ion channels (channelopathies)**

| **AFFECTED TISSUE** | | | |
|---|---|---|---|
| **skeletal muscle** | **cardiac muscle** | **CNS** | **Others** |
| **Ataxias** | Long QT syndrome 5 (KCNE1), | cortical hyperexcitability | **Auditory disorders:** Dominant deafness |
| Episodic ataxia 2 (CACNA1A) | | **epilepsy syndromes** | (KCNQ4) |
| | Jervell-Lange-Nielsen Lange-Nielsen Syndrome (KCNE1, KCNQ1), | and Generalized epilepsy | **Hyperthermia:** |
| spinocerebellar ataxia 6 (CACNA1A) | | (CACNB4) Benign familial neonatal | Malignant hyperthermia 5 (CACNA1S), |
| KCNA1 (potassium channel) episodic ataxia | | convulsions 1 (KCNQ2) Benign familial neonatal | malignant hyperthermia 1 |
| **Myotonias** | inducible long QT | convulsions 2 (KCNQ3) | (RYR1) |
| CLCN1 (chloride channel) e.g. Thomsen myotonia, Becker myotonia, myotonia congenital, generalized myotonia, myotonia levior | syndrome (KCNE2) | nocturnal frontal lobe epilepsy (CHRNA4) | Central core disease (RYR1) |
| | long QT syndrome 1 (KCNQ1) | *(Lehmann-Horn* & *Jurkat-* | **Renal disorders:** Polycystic kidney disease (PKD1), Dent's disease (CLCN5), Bartter syndrome (CLCNKB), |
| | long QT syndrome 2 (hERG) | *Rott, 2000)* | |
| **Myasthenias** | | | |
| KCNQ2 (potassium channel) myokymia | | | |
| Hypokalemic periodic paralysis 1 (CACNA1 S) | | | |

**Table 2c. Other diseases due to hyperexcitable tissue (Hyperexcitability Disorders)**

| **AFFECTED TISSUE** | |
|---|---|
| **CNS** | **PNS** |
| Post traumatic stress disorder *(Centonze et al. 2005)* | Peripheral nerve hyperexcitability syndromes |
| Alzheimer's disease, Parkinson's disease, Bipolar disorder and Schizophrenia | |
| *(Farber et al. 2002)* | |

### References:

Farber, NB et al., Molecular Psychiatry (2002), 7:726-733Lehmann-Horn, Jurkat-Rott www.channelopathies.org
Meisler MH, Kearney JA. J Clin Invest. 2005 Aug;115(8):2010-7. PMID: 16075041
Siep E, et al. Neurobiol Dis. 2002 Mar;9(2):258-68.
Victor M, Ropper AH Adams and Victor's Principles of Neurology. 7th edition, McGraw-Hill New York 2001.
Yang Y et al. J Med Genet. 2004 Mar;41(3):171-4.

Based on the finding that the compounds of the present invention are capable of increasing slow inactivation of voltage-gated sodium channels and thereby act against hyperexcitability, it is concluded that the pharmaceutical composition of the present invention is suitable for the treatment of diseases associated with hyperexcitability, such as anxiety or/and stress.

Thus, in another preferred embodiment of the present invention, the disease associated with hyperexcitability is a disease selected from anxiety, stress, posttraumatic stress disorder, and peripheral nerve hyperexcitability syndromes. In an even more preferred embodiment of the present invention, the disease is stress-induced anxiety as for instance seen in post-traumatic stress disorder.

Hyperexcitability may cause neurodegeneration. Example 3 of the present invention demonstrates a neuroprotective effect of the compounds of the present invention. Thus, in a preferred embodiment, the prevention, alleviation or/and treatment of a disease associated with hyperexcitability is effected by neuroprotection, in particular by short-term neuroprotection. In this preferred embodiment of the present invention, the disease associated with hyperexcitability is a condition associated with neuronal damage or/and neurodegeneration. More preferably, the disease associated with hyperexcitability is a condition associated with neuronal damage or/and neurodegeneration caused by a neurodegenerative disease or/and a psychotic disease. The neurodegenerative disease or/and the psychotic disease is preferably selected from Alzheimer's disease, Parkinson's disease, bipolar disorder, and schizophrenia.

The disease associated with hyperexcitability may not be epilepsy, status epilepticus, pain, neuropathic pain, allodynia or hyperalgesia. The disease associated with hyperexcitability may not be an epilepsy related condition or a neuropathic pain related condition. It shall be understood that the specific forms of epilepsy and pain as described herein may not be subject of these disclaimers.

The disease associated with hyperexcitability may not be amyotrophic lateral sclerosis or Guillain-Barré syndrome. It shall be understood that paralysis associated with amyotrophic lateral sclerosis or/and Guillain-Barré syndrome as described herein may not be subject of this disclaimer.

The disease associated with hyperexcitability may not be Alzheimer's disease, Parkinson's disease, bipolar disorder, or schizophrenia. It shall be understood that a condition associated with neuronal damage or/and neurodegeneration caused by a neurodegenerative disease or/and psychotic disease preferably selected from Alzheimer's disease, Parkinson's disease, bipolar disorder, and schizophrenia may not be subject of this disclaimer.

The disease associated with hyperexcitability may not be tinnitus or obsessive compulsive disorder.

The compounds of the present invention of Formulae (I), (II) or/and (ill), in particular lacosamide, are well tolerated, which is an advantage over other commonly used therapeutics for treatment of diseases associated with hyperexcitability.

The compounds of the present invention, in particular lacosamide, may be used in a first line treatment of a disease as defined herein. Therefore, the pharmaceutical composition of the present invention is suitable for a first line treatment of a disease as defined herein.

The compounds of the present invention may also be used in a second line treatment of a disease as defined herein. Therefore, the pharmaceutical composition of the present invention is also suitable for a second line treatment of a disease as defined herein.

Yet another aspect of the present invention is a pharmaceutical composition comprising at least one compound of Formulae (I), (II), or/and (III) as defined herein, preferably lacosamide, for the prevention, alleviation or/and treatment of a disease associated with hyperexcitability, as defined herein.

The components of Formulae (I), (II), or/and (III) may also be administered together with a further active agent for the treatment of a disease associated with hyperexcitability, as defined herein.

A further aspect of the present invention is a pharmaceutical composition comprising
(a) at least one compound of Formulae (I), (II), or/and (III) as defined herein, preferably lacosamide, and
(b) at least one further active agent for the prevention, alleviation, or/and treatment of a disease associated with hyperexcitability as defined herein.

The compound of Formulae (I), (II), or/and (III) and the further active agent (b) may be formulated in one pharmaceutical preparation (single dosage form) for administration at the same time or may be formulated in two or more distinct preparations (separate dosage forms) for simultaneous or/and subsequent administration. The two distinct preparations in the separate dosage forms may be administered by the same route or by different routes.

Separate dosage forms can optionally be co-packaged, for example in a single container or in a plurality of containers within a single outer package, or co-presented in separate packaging ("common presentation"). As an example of co-packaging or common presentation, a kit is contemplated comprising, in separate containers, compound of Formulae (I), (II), or/and (III) and the further active agent (b). In another example, the compound of Formulae (I), (II), or/and (III) and the further active agent (b) are separately packaged and available for sale independently of one another, but are co-marketed or co-promoted for use according to the invention. The separate dose forms may also be presented to a subject separately and independently, for use according to the invention.

In a further preferred embodiment, the pharmaceutical composition comprises a single dosage form comprising at least one compound of Formulae (I), (II), or/and (III) and at least one further active agent (b).

In another preferred embodiment, the pharmaceutical composition of the present invention comprises separate dosage forms comprising
(i) a first composition comprising at least one compound of Formulae (I), (II), or/and (III), and
(ii) a second composition comprising at least one further active agent for the prevention, alleviation, or/and treatment of disease associated with hyperexcitability, as defined herein.

In yet another preferred embodiment of the present invention, the second composition (ii) comprising the at least one further active agent may be a commercially available composition.

The pharmaceutical composition of the present invention is preferably prepared for administration in mammals, preferably in humans.

The pharmaceutical composition of the present invention comprising (a) at least one compound of Formulae (I), (II) or/and (III) and (b) at least one further active agent may be prepared for the prevention, alleviation or/and treatment of disease associated with hyperexcitability, as described herein.

Yet another aspect of the present invention is a method for the prevention, alleviation or/and treatment of a disease associated with hyperexcitability, wherein the method comprises administering to a subject in need thereof at least one compound of Formulae (I), (II), or/and (III), in particular lacosamide.

A further aspect of the present invention is a method for the prevention, alleviation or/and treatment of a disease associated with hyperexcitability, wherein the method comprises co-administering to a subject in need thereof at least one compound of Formulae (I), (II), or/and (III), in particular lacosamide, and a further active agent for the prevention, alleviation, or/and treatment of a disease associated with hyperexcitability in therapeutically effective amounts.

Especially preferred is the co-administration of at least one compound of formulae (I), (II), or/and (III), in particular lacosamide, and mexiletine, carbonic anhydrase inhibitors (such as acetazolamide or dichlorphenamide), benzodiazepines (such as diazepam, midazolam, alprazolam), SSRIs (selective serotonin reuptake inhibitors such as fluoxetine, fluvoxamine, sertraline, venlafaxine, mirtazapine), baclofen, quinine, phenytoin and other anticonvulsant drugs (such as lamotrigine, levetiracetam, topiramate, carbamazepine, oxcarbazepine, tiagabine, vigabatrine, zonisamide), tricyclic antidepressants (such as amitryptiline, imipramine, desipramine), nonsteroidal antiinflammatory drugs (NSAIDs such as acetylsalicylic acid, paracetamol, ibuprofen, naproxen), acetylcholinesterase inhibitors (AChE) inhibitors (such as tacrine, rivastigmine, galanthamine, donezepil), dopa decarboxylase inhibitors, dopamine agonists (such as ropinirole, pramipexole, lisuride, pergolide, piribedil), monoamine oxidase-B and COMT inhibitors (such as tolcapone, entacapone, apomorphine, rasagiline), atypical antipsychotic medications (such as clozapine, risperidone, olanzapine, quetiapine, ziprasidone, aripiprazole, and amisulpride), or potassium tablets.

The term "co-administration" refers to a plurality of agents that, when administered to a subject together or separately, are co-active in bringing therapeutic benefit to the subject. Such co-administration is also referred to as "combination", "combination therapy," "co-therapy," "adjunctive therapy" or "add-on therapy." For example, one agent can enhance the therapeutic effect of another, or reduce an adverse side effect of another, or one or more agents can be effectively administered at a lower dose than when used alone, or can provide greater therapeutic benefit than when used alone, or can complementarily address different aspects, symptoms or etiological factors of a disease or condition.

Co-administration comprises administration of the combination of the agents in amounts sufficient to achieve or/and maintain therapeutically effective concentrations, e.g. plasma concentrations, in the subject in need thereof. Co-administration comprises simultaneous or/and subsequent administration. Simultaneous administration comprises administration of the agents as a single or as different compositions.

The administration interval of the compound of Formulae (I), (II), or/and (III) and the further active agent may depend on the dosage forms. The compound of Formulae (I), (II), or/and (III) may be administered first, or the further active agent (b) may be administered first.

The compound according to the invention has the general Formula (I) 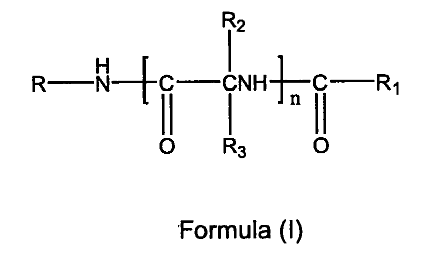 wherein
R is hydrogen, alkyl, alkenyl, alkynyl, aryl, aryl alkyl, heterocyclic, heterocyclic alkyl, alkyl heterocyclic, cycloalkyl or cycloalkyl alkyl, and R is unsubstituted or is substituted with at least one electron withdrawing group, or/and at least one electron donating group;
R₁ is hydrogen or alkyl, alkenyl, alkynyl, aryl alkyl, aryl, heterocyclic alkyl, alkyl heterocyclic, heterocyclic, cycloalkyl, cycloalkyl alkyl, each unsubstituted or substituted with at least one electron donating group or/and at least one electron withdrawing group;
   and
R₂ and R₃ are independently hydrogen, alkyl, alkenyl, alkynyl, alkoxy, alkoxyalkyl, aryl alkyl, aryl, halo, heterocyclic, heterocyclic alkyl, alkyl heterocyclic, cycloalkyl, cycloalkyl alkyl, or Z-Y wherein R₂ and R₃ may be unsubstituted or substituted with at least one electron withdrawing group or/and at least one electron donating group;
Z is O, S, S(O)ₐ, NR₄, NR'₆, PR₄ or a chemical bond;
Y is hydrogen, alkyl, aryl, aryl alkyl, alkenyl, alkynyl, halo, heterocyclic, heterocyclic alkyl, alkyl heterocyclic and Y may be unsubstituted or substituted with at least one electron donating group or/and at least one electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 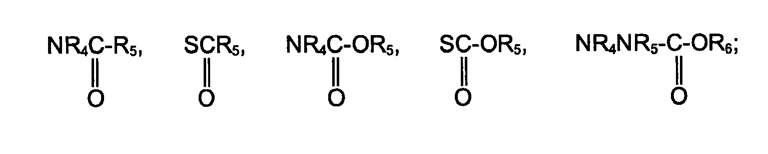
R'₆ is hydrogen, alkyl, alkenyl, or alkenyl which may be unsubstituted or substituted with at least one electron withdrawing group or/and at least one electron donating group;
R₄, R₅ and R₆ are independently hydrogen, alkyl, aryl, aryl alkyl, alkenyl, or alkynyl, wherein R₄, R₅ and R₆ may independently be unsubstituted or substituted with at least one electron withdrawing group or/and at least one electron donating group;
R₇ is R₆ or COOR₈ or COR₈, which R₇ may be unsubstituted or substituted with at least one electron withdrawing group or/and at least one electron donating group;
R₈ is hydrogen or alkyl, or aryl alkyl, and the aryl or alkyl group may be unsubstituted or substituted with at least one electron withdrawing group or/and at least one electron donating group; and
n is 1-4; and
a is 1-3.

In a preferred embodiment, the compound of Formula (I) has the general Formula (II), 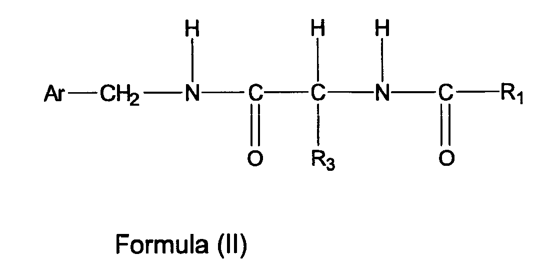 wherein
Ar is aryl which is unsubstituted or substituted with at least one electron donating group or/and at least one electron withdrawing group, preferably halo, more preferably fluoro;
R₁ is alkyl, preferably alkyl containing 1-3 carbon atoms, more preferably methyl; and
R₃ is as defined herein.

In a more preferred embodiment, the compound of Formulae (I) or/and (II) has the general Formula (III), 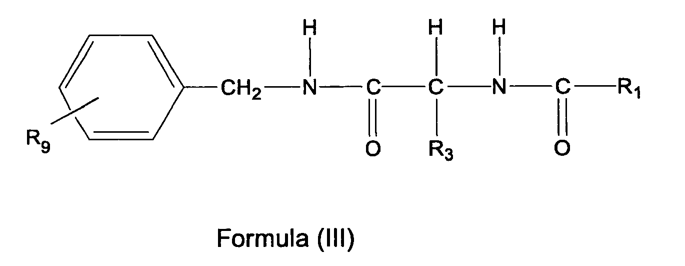 wherein
R₉ is one or more substituents independently selected from the group consisting of hydrogen, halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide;
R₃ is selected from the group consisting of hydrogen, alkyl, arylalkyl, alkoxy, alkoxyalkyl, aryl, heterocyclic, heterocyclic alkyl, N-alkoxy-N-alkylamino, N-alkoxyamino, and N-carbalkoxy; and
R₁ is alkyl, preferably alkyl containing 1 to 3 carbon atoms, more preferably methyl.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R⁻ or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

As used herein, the term configuration particularly refers to the configuration around the carbon atom to which R₂ and R₃ or H and R₃ are attached, even though other chiral centers may be present in the molecule. Therefore, when referring to a particular configuration, such as D or L, it is to be understood to mean the D or L stereoisomer at the carbon atom to which R₂ and R₃ or H and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ or H and R₃ are attached). These stereoisomers may be found in mixtures of the L and D stereoisomer, e.g., racemic mixtures. The D stereoisomer is preferred.

It is preferred that the compounds of Formula (I) are in the R configuration. It is also preferred that the compounds of Formula (II) are in the R configuration. It is also preferred that the compounds of Formula (III) are in the R configuration.

It is preferred that the compounds of Formulae (I), (II) or/and (III) in the R configuration are substantially enantiopure. As used herein, the term "substantially enantiopure" refers to a content of the R enantiomer of at least 99.5%. This corresponds to an enantiomeric excess (ee) of 99%. The respective quantities of R and S enantiomer may be determined by chiral column chromatography, e.g. by HPLC with "ChiralPak" as chiral, stationary phase.

The term "alkyl" (alone or in combination with another term(s)) means a straight- or branched-chain saturated hydrocarbyl substituent preferably containing from 1 to about 20 carbon atoms (C₁-C₂₀-alkyl), more preferably from 1 to about 8 carbon atoms (C₁-C₈-alkyl), even more preferably from 1 to about 6 carbon atoms (C₁-C₆-alkyl), and most preferably from 1 to 3 carbon atoms (C₁-C₃-alkyl). The alkyl groups include methyl, ethyl, propyl, isopropyl, butyl, isobutyl, tertiary butyl, amyl, hexyl, and the like. Further, alkyl groups also include halogenated alkyl groups up to perhalogenation, e.g. trifluoromethyl, if not indicated otherwise.

The term "alkoxy" (alone or in combination with another term(s)) refers to -O-alkyl and means a straight- or branched-chain alkoxy substituent preferably containing from 1 to about 20 carbon atoms (C₁-C₂₀-alkoxy), more preferably from 1 to about 8 carbon atoms (C₁-C₈-alkoxy), even more preferably from 1 to about 6 carbon atoms (C₁-C₆-alkoxy), and most preferably from 1 to 3 carbon atoms (C₁-C₃-alkoxy). The alkoxy groups include methoxy, ethoxy, propoxy, butoxy, isobutoxy, tert-butoxy, pentoxy, hexoxy and the like. Further, alkoxy groups include halogenated alkoxy groups up to perhalogenation, if not indicated otherwise.

The term "alkoxyalkyl" refers to an alkyl group substituted with at least one alkoxy group. The alkoxyalkyl groups include methoxymethyl (-CH₂-OCH₃) groups, methoxyethyl (-CH₂-CH₂-OCH₃) groups, ethoxymethyl (-CH₂-O-CH₂CH₃) groups and the like.

The term "N-alkoxyamino" refers to amino groups substituted with one or two alkoxy groups, e.g. -NH-N(OCH₃)₂.

The term "N-alkoxy-N-alkylamino" refers to amino groups substituted with an alkoxy group and an alkyl group, e.g. -N(CH₃)(OCH₃), -N(CH₃)(OCH₂-CH₃) and the like.

The term "N-carbalkoxy" refers to amino groups substituted with a carbalkoxy group, e.g. -NH(C(O)-O-CH₃), -NH(C(O)O-CH₂-CH₃).

The term "aryl", when used alone or in combination with other term(s), refers to an aromatic group which contains from 6 up to 18 ring carbon atoms (C₆-C₁₈-aryl), preferably from 6 up to 10 ring carbon atoms (C₆-C₁₀-aryl), and includes polynuclear aromatics. The aryl groups may be monocyclic, bicyclic, tricyclic or polycyclic and may be fused rings. A polynuclear aromatic compound as used herein, is meant to encompass bicyclic and tricyclic fused aromatic ring systems containing from 10-18 ring carbon atoms. Aryl groups include phenyl and polynuclear aromatics e.g., naphthyl, anthracenyl, phenanthrenyl, azulenyl and the like. The aryl group also includes groups such as ferrocenyl. Aryl groups may be unsubstituted or mono or polysubstituted with electron withdrawing or/and electron donating groups. A preferred aryl group is phenyl, which may unsubstituted or mono or polysubstituted with electron withdrawing or/and electron donating groups.

The term "aryl alkyl" as used herein alone or in combination with other term (s) means an alkyl group as defined herein carrying an aryl substitutent as defined herein. Preferred aryl alkyl groups are aryl-C₁-C₆-alkyl, aryl-C₁-C₃-alkyl, C₆-C₁₀-aryl-alkyl, C₆-C₁₀-aryl-C₁-C₆-alkyl, C₆-C₁₀-aryl-C₁-C₃-alkyl. More preferred aryl alkyl groups are phenyl-C₁-C₆-alkyl and phenyl-C₁-C₃-alkyl. Even more preferred aryl alkyl groups include, for example, benzyl, phenylethyl, phenylpropyl, phenylisopropyl, phenylbutyl, diphenylmethyl, 1,1-diphenylethyl, 1,2-diphenylethyl, and the like. Most preferred is benzyl.

The term "alkenyl" (alone or in combination with another term(s)) means a straight- or branched-chain alkenyl substituent containing at least one double bond and preferably containing from 2 to about 20 carbon atoms (C₂-C₂₀-alkenyl), more preferably from 2 to about 8 carbon atoms (C₂-C₈-alkenyl), and even more preferably from 2 to about 6 carbon atoms (C₂-C₆-alkenyl), most preferably 2 or 3 carbon atoms (C₂-C₃-alkenyl). The alkenyl group may be in the Z or E form. Alkenyl groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "alkynyl" (alone or in combination with another term(s)) means a straight- or branched-chain alkynyl substituent containing at least one triple bond and preferably containing from 2 to about 20 carbon atoms (C₂-C₂₀-alkynyl), more preferably from 2 to about 8 carbon atoms (C₂-C₈-alkynyl), and even more preferably from 2 to about 6 carbon atoms (C₂-C₆-alkynyl), most preferably 2 or 3 carbon atoms (C₂-C₃-alkynyl). The alkynyl group includes ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "cycloalkyl" when used alone or in combination with another term (s) means a cycloalkyl group containing from 3 to 18 ring carbon atoms (C₃-C₁₈-cycloalkyl), preferably from 6 up to 10 ring carbon atoms (C₃-C₁₀-cycloalkyl). The cycloalkyl groups may be monocyclic, bicyclic, tricyclic, or polycyclic, and the rings may be fused. The cycloalkyl may be completely saturated or partially saturated. Examples of cycloalkyl groups include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. The cycloalkyl group includes the cis or trans forms. Cycloalkyl groups may be unsubstituted or mono or polysubstituted with electron withdrawing or/and electron donating groups. In a bridged bicyclic cycloalkyl group, the substituents may either be in endo or exo positions.

The term "cycloalkyl alkyl" as used herein alone or in combination with other term(s) means an alkyl group as defined herein carrying a cycloalkyl substitutent as defined herein. Preferred cycloalkyl alkyl groups are cycloalkyl-C₁-C₆-alkyl, cycloalkyl-C₁-C₃-alkyl, C₆-C₁₀-cycloalkyl-alkyl, C₆-C₁₀-cycloalkyl-C₁-C₆-alkyl, C₆-C₁₀-cycloalkyl-C₁-C₃-alkyl. A more preferred cycloalkyl alkyl group is selected from cyclohexyl-C₁-C₆-alkyl and cyclohexyl-C₁-C₃-alkyl.

The term "halo" or "halogen" includes fluoro, chloro, bromo, and iodo.

The prefix "halo" indicates that the substituent to which the prefix is attached is substituted with one or more independently selected halogen radicals. For example, haloalkyl means an alkyl substituent wherein at least one hydrogen radical is replaced with a halogen radical. Examples of haloalkyls include chloromethyl, 1-bromoethyl, fluoromethyl, difluoromethyl, trifluoromethyl, 1,1,1-trifluoroethyl, and the like. Illustrating further, "haloalkoxy" means an alkoxy substituent wherein at least one hydrogen radical is replaced by a halogen radical. Examples of haloalkoxy substituents include chloromethoxy, 1-bromoethoxy, fluoromethoxy, difluoromethoxy, trifluoromethoxy (also known as "perfluoromethyloxy"), 1,1,1,-trifluoroethoxy, and the like. It should be recognized that if a substituent is substituted by more than one halogen radical, those halogen radicals may be identical or different (unless otherwise stated).

The terms "electron-withdrawing" and "electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo; nitro, carboxy, alkenyl, alkynyl, formyl, carboxyamido, aryl, quaternary ammonium, haloalkyl such as trifluoromethyl, aryl alkanoyl, carbalkoxy and the like. Electron donating groups include such groups as hydroxy, alkoxy, including methoxy, ethoxy and the like; alkyl, such as methyl, ethyl, and the like; amino, alkylamino, dialkyl amino, aryloxy such as phenoxy, mercapto, alkylthio, alkylmercapto, disulfide (alkyldithio) and the like. One of ordinary skill in the art will appreciate that some of the aforesaid substituents may be considered to be electron donating or electron withdrawing- under different chemical conditions. Moreover, the present invention contemplates any combination of substituents selected from the above-identified groups.

The electron donating or/and electron withdrawing groups may independently be present in any one of the substituents in Formula (I), (II) or/and (III) e.g., in R, R₁, R₂, R₃, R₄, R₅, R₆, R'₆, R₇, R₈, R₉ or/and Rio as defined herein.

The at least one electron withdrawing or/and at least one electron donating group is preferably selected independently from halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, disulfide, alkanoyl, amino alkyl, aryloyl, cyano, sulfonyl, sulfoxide, heterocyclic, guanidine, sulfonium salts, mercaptoalkyl, and alkyldithio.

The term "sulfide" encompasses mercapto, mercapto alkyl and alkylthio, while the term disulfide encompasses alkyldithio.

In the compounds of the present invention, the at least one electron withdrawing or/and at least one electron donating group is more preferably selected independently from halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide.

Even more preferably, the at least one electron withdrawing or/and at least one electron donating group is selected from halo, C₁-C₆-alkyl, C₂-C₆-alkenyl, C₁-C₆-alkynyl, nitro, carboxy, formyl, carboxyamido, C₆-C₁₀-aryl, quaternary ammonium, C₁-C₆-haloalkyl, C₆-C₁₀-aryl C₂-C₆-alkanoyl, hydroxy, C₁-C₆-alkoxy, C₂-C₆-carbalkoxy, amino, C₁-C₆-alkylamino, C₁-C₆-dialkylamino, C₆-C₁₀-aryloxy, mercapto, C₁-C₆-alkylthio, C₁-C₆-alkylmercapto, and disulfide.

Even more preferably, the electron withdrawing or/and electron donating groups may also be independently selected from halo, C₁-C₆-alkoxy, nitro, carboxy, formyl, carboxyamido, quaternary ammonium, hydroxy, amino, mercapto, and disulfide.

Most preferred electron withdrawing or/and electron donating groups are independently selected from halo such as fluoro and C₁-C₆-alkoxy such as methoxy and ethoxy.

The term "carbalkoxy" as used herein alone or in combination with other term(s) means an -CO-O-alkyl, wherein alkyl is as defined herein, taking into account that the -CO-O- group provides one carbon atom in addition to those of the alkyl group. The carbalkoxy group preferably contains from 2 to about 20 carbon atoms (C₂-C₂₀-carbalkoxy), more preferably from 2 to about 8 carbon atoms (C₂-C₈-carbalkoxy), even more preferably from 2 to about 6 carbon atoms (C₂-C₆-carbalkoxy), and most preferably from 2 to 3 carbon atoms (C₂-C₃-carbalkoxy).

The term "alkanoyl" as used herein alone or in combination with other term (s) means an alkanoyl group -CO-alkyl, wherein alkyl is as defined herein, taking into account that the -CO- group provides one carbon atom in addition to those of the alkyl group. The alkanoyl preferably contains from 2 to about 20 carbon atoms (C₂-C₂₀-alkanoyl), more preferably from 2 to about 8 carbon atoms (C₂-C₈-alkanoyl), even more preferably from 2 to about 6 carbon atoms (C₂-C₆-alkanoyl), and most preferably from 2 to 3 carbon atoms (C₂-C₃-alkanoyl). The alkanoyl group may be straight chained or branched. The alkanoyl groups include, for example, formyl, acetyl, propionyl, butyryl, isobutyryl, tertiary butyryl, pentanoyl and hexanoyl.

As employed herein, a heterocyclic group contains at least one heteroatom in the cyclic structure, preferably one, two, three or four heteroatoms. The at least one heteroatom may be independently selected from sulfur, nitrogen and oxygen. The heterocyclic groups contemplated by the present invention include heteroaromatics and saturated and partially saturated heterocyclic groups. The heterocyclics may be monocyclic, bicyclic, tricyclic or polycyclic and may be fused rings. The heterocyclics also include the so-called benzoheterocyclics. Heterocyclic groups may be unsubstituted or mono or polysubstituted with electron withdrawing or/and electron donating groups. The heterocyclic groups preferably contain up to 18 ring atoms and up to a total of 17 ring carbon atoms and may be unsubstituted or mono or polysubstituted with electron withdrawing or/and electron donating groups.

More preferably, the heterocyclic group may be independently selected from 5 or 6-membered monocyclic heterocyclic groups and may be unsubstituted or mono or polysubstituted with electron withdrawing or/and electron donating groups. The heterocyclic group may also be more preferably selected independently from furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like. Even more preferably, the heterocyclic moieties are those aforementioned heterocyclics which are monocyclic.

The heterocyclics may also be more preferably selected independently from thienyl, furyl, pyrrolyl, benzofuryl, benzothienyl, indolyl, oxazolyl, methylpyrrolyl, morpholinyl, pyridiyl, pyrazinyl, imidazolyl, pyrimidinyl, and pyridazinyl. Especially preferred heterocyclic are independently selected from furyl, oxazolyl, pyridyl, pyrazinyl, imidazolyl, pyrimidinyl, and pyridazinyl. The most preferred heterocyclics are independently selected from furyl, pyridyl and oxazolyl.

The monocyclic 5- or 6-membered heterocyclic groups in the compounds of the present invention are preferably of the Formula (IV): 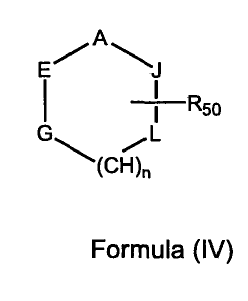 or those corresponding partially or fully saturated form thereof, wherein n is 0 or 1; and
R₅₀ is H, an electron withdrawing group or an electron donating group;
A, E, L, J and G are independently CH, or a heteroatom selected from the group consisting of N, O, S; but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety.

If the ring depicted in Formula (IV) contains a nitrogen ring atom, then the N-oxide forms are also contemplated to be within the scope of the invention.

When R₂ or R₃ is a heterocyclic of Formula (IV), it may be bonded to the main chain by a ring carbon atom. When n is 0, R₂ or R₃ may additionally be bonded to the main chain by a nitrogen ring atom.

The term "heterocyclic alkyl" as used herein alone or in combination with other term(s) means an alkyl group as defined above carrying a heterocyclic substituent as defined above. Preferred heterocyclic alkyl groups are heterocyclic-C₁-C₆-alkyl, heterocyclic-C₁-C₃-alkyl, wherein the heterocyclic may be a preferred, more preferred or most preferred heterocyclic group as defined herein.

The term "alkyl heterocyclic" as used herein alone or in combination with other term(s) means a heterocyclic group as defined above carrying at least one alkyl substituent as defined above. Preferred alkyl heterocyclic groups are C₁-C₆-alkyl-heterocyclic, C₁-C₃-alkyl-heterocyclic, wherein the heterocyclic group may be a preferred, more preferred or most preferred heterocyclic group as defined herein.

The preferred compounds are those wherein n is 1, but di (n=2), tri (n=3) and tetrapeptides (n=4) are also contemplated to be within the scope of the invention.

In the ZY groups representative of R₂ or/and R₃, in the formula (I) or/and (II), Z may be O, S, S(O)ₐ, wherein a is 1-3, NR₄, NR'₆, PR₄ or a chemical bond; and Y may be hydrogen, alkyl, aryl, aryl alkyl, alkenyl, alkynyl, halo, heterocyclic, heterocyclic alkyl, alkyl heterocyclic, and Y may be unsubstituted or substituted with at least one electron donating group or/and at least one electron withdrawing group, provided that when Y is halo, Z is a chemical bond, or
ZY taken together may be NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 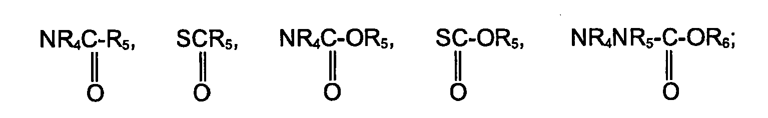 wherein R₄, R₅, R'₆, R₆, R₇, are as defined herein.

The ZY groups representative of R₂ or/and R₃ in the Formula (I) or/and (II) may be hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; dialkylamino, such as, dimethylamino; trialkyl ammonium salt; hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), alkoxy amino [(NHOR₁₈) wherein R₁₈ is alkyl], N-alkylhydroxyl amino [(NR₁₈)OH wherein R₁₈ is alkyl], N-alkyl-O-alkylhydroxyamino, i.e., [N(R₁₈)OR₁₉ wherein R₁₈ and R₁₉ are independently alkyl], and O-hydroxylamino (-O-NH₂); alkylamido such as acetamido; trifluoroacetamido; alkoxyamino, (e.g., NH(OCH₃); and heterocyclicamino, such as pyrazoylamino.

In a preferred ZY group, Z is O, NR₄ or PR₄; Y is hydrogen or alkyl.

In another preferred embodiment,

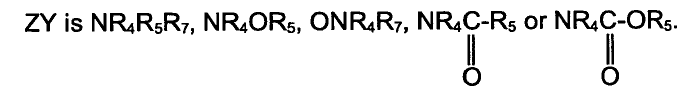

In a more preferred that ZY is NR₄OR₅, or ONR₄R₇.

Another more preferred ZY is N-hydroxyamino, N-alkylhydroxyamino, N-alkyl-O-alkyl hydroxyamino, O-alkylhydroxyamino, N-alkoxy-N-alkylamino, N-alkoxyamino, or N-carbalkoxy.

In Formula (I), R is preferably aryl or aryl alkyl, more preferably R is aryl alkyl, wherein R is unsubstituted or substituted with at least one electron donating group or/and at least one electron withdrawing group. R may be phenyl or benzyl, most preferably benzyl, wherein R is unsubstituted or substituted with at least one electron donating group or/and at least one electron withdrawing group. If R is substituted, R is preferably substituted on the aryl ring. In this embodiment, the at least one electron donating group or/and at least one electron withdrawing group is preferably halo, more preferably fluoro.

In Formulae (I), (II) or/and (III), R₁ is H or alkyl. More preferably, R₁ is alkyl, preferably containing from 1 to 6 carbon atoms, more preferably containing from 1 to 3 carbon atoms. Most preferably the R₁ group is methyl. R₁ may be unsubstituted or substituted with at least one electron donating group or/and at least one electron withdrawing group.

Further, it is preferred that one of R₂ and R₃ is hydrogen. It is more preferred that R₂ is hydrogen. Other preferred moieties of R₂ in Formula (I) are aryl such as phenyl, aryl alkyl such as benzyl, and alkyl. It is to be understood that the preferred groups of R₂ may be unsubstituted or mono or poly substituted with electron donating or/and electron withdrawing groups. It is preferred that the at least one electron withdrawing or/and at least one donating group in R₂ is independently alkoxy, N-hydroxyamino, N-alkylhydroxyamino, N-alkyl-O-alkyl hydroxyamino or O-alkylhydroxyamino, and especially methoxy or ethoxy.

In Formulae (I), (II) or/and (III), R₃ may be hydrogen, an alkyl group unsubstituted or substituted by at least an electron donating or/and at least one electron withdrawing group, an aryl group unsubstituted or substituted by at least an electron donating or/and at least one electron withdrawing group heterocyclic, heterocyclic alkyl, or ZY.

It is preferred that R₃ is hydrogen, alkyl unsubstituted or substituted by at least an electron donating or/and at least one electron withdrawing group, aryl which is unsubstituted or substituted by at least one electron donating group or/and at least one electron withdrawing group, heterocyclic, heterocyclic alkyl or ZY, wherein Z is O, NR₄ or PR₄; Y is hydrogen or alkyl;

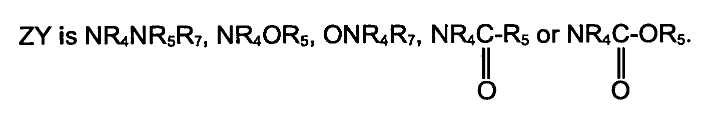

It is also preferred that R₃ is alkyl unsubstituted or substituted by at least an electron donating or/and at least one electron withdrawing group; or Z-Y, wherein Z-Y is as defined herein.

It is also preferred that R₃ is alkyl unsubstituted or substituted by at least an electron donating or/and at least one electron withdrawing group; NR₄OR₅, or ONR₄R₇, wherein R₄, R₅ and R₇ are as defined herein.

It is also preferred that R₃ is CH₂-Q, wherein Q is alkoxy especially containing 1-3 carbon atoms; or R₃ is NR₄OR₅ or ONR₄R₇, wherein R₄, R₅, and R₇ are as defined herein.

R₃ is also preferably alkyl which is unsubstituted or substituted with at least one alkoxy especially containing 1-3 carbon atoms.

R₃ is also preferably CH₂-Q, wherein Q is alkoxy preferably containing 1-3 carbon atoms, more preferably Q is ethoxy or methoxy.

R₃ is also preferably NR₄OR₅, or ONR₄R₇, wherein R₄, R₅ and R₇ are as defined herein, and R₄, R₅ and R₇ are as defined herein, e.g. N-alkoxy, N-alkoxy-N-alkylamino or N-carbalkoxy.

R₃ is also preferably heterocyclic, heterocyclic alkyl, or aryl, which may be unsubstituted or substituted with at least an electron donating or/and at least one electron withdrawing group. A most preferred heterocyclic in R₃ is furyl or oxazolyl.

R₃ is also preferably selected from the group consisting of hydrogen, alkyl, arylalkyl such as benzyl, alkoxy, alkoxyalkyl, aryl such as phenyl, heterocyclic, heterocyclic alkyl, N-alkoxy-N-alkylamino, N-alkoxyamino and N-carbalkoxy.

It is to be understood that the preferred groups of R₃ may be unsubstituted or mono or poly substituted with electron donating or/and electron withdrawing groups. It is preferred that the at least one electron withdrawing or/and at least one electron donating group in R₃ is independently alkoxy, N-hydroxyamino, N-alkylhydroxyamino, N-alkyl-O-alkyl hydroxyamino or O-alkylhydroxyamino, and especially methoxy or ethoxy.

R₄, R₅, R₆, R'₆, R₇ and R₈ are preferably independently hydrogen or alkyl.

R₄, R₅, and R₇ are preferably independently hydrogen or alkyl preferably containing 1-3 carbon atoms.

The most preferred aryl is phenyl. The most preferred halo is fluoro.

In the compounds of Formula (I), R is preferably aryl alkyl, wherein R is unsubstituted or substituted with at least one electron donating group or/and at least one electron withdrawing group.

In the compounds of Formula (I), R₁ is preferably alkyl which is unsubstituted or substituted with at least one electron donating group or/and at least one electron withdrawing group.

In the compounds of Formula (I), R₂ and R₃ is preferably independently hydrogen, alkyl which is unsubstituted or substituted by at least one electron donating group or/and at least one electron withdrawing group, aryl which is unsubstituted or substituted by at least one electron donating group or/and at least one electron withdrawing group, heterocyclic, heterocyclic aryl, or ZY; wherein Z is O, NR₄ or PR₄; and Y is hydrogen or 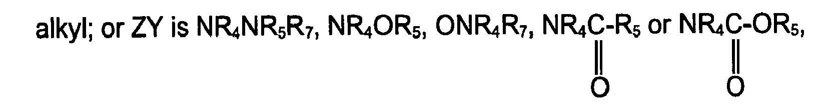 wherein R₄, R₅ and R₇ are as defined herein.

In the compounds of Formula (I), the preferred groups of R₂ and R₃ may be unsubstituted or mono or poly substituted with -electron donating or/and electron withdrawing groups, such as alkoxy (e.g., methoxy, ethoxy, and the like), N-hydroxyamino, N-alkylhydroxyamino, N-alkyl-O-alkyl hydroxyamino and O-alkylhydroxyamino.

In the compounds of Formula (I), the at least one electron donating group or/and at least one electron withdrawing group in R₂ or/and R₃ is preferably independently hydroxy or alkoxy.

It is more preferred that in the compounds of Formula (I), R₂ is hydrogen.

In the compounds of Formula (II), R₁ is preferably methyl.

In preferred compounds of Formula (II), R₃ is hydrogen or alkyl unsubstituted or substituted by at least one electron donating group or/and at least one electron withdrawing group; or R₃ is heterocyclic, heterocyclic alkyl, or Z-Y, wherein Z-Y and heterocyclic are as defined herein.

In other preferred compounds of Formula (II), R₃ is an alkyl group which is unsubstituted or substituted by at least one electron donating group or/and at least one electron withdrawing group, NR₄OR₅ or ONR₄R₇, wherein R₄, R₅ and R₇ are as defined herein and wherein the at least one electron donating group or/and at least one electron withdrawing group is preferably selected from hydroxy and alkoxy.

In further preferred compounds of Formula (II), R₃ is CH₂-Q, wherein Q is alkoxy preferably containing 1-3 carbon atoms, more preferably methoxy, or R₃ is NR₄OR₅ or ONR₄R₇ wherein R₄, R₅ and R₇ are independently hydrogen or alkyl containing 1-3 carbon atoms.

In other preferred compounds of Formula (II), R₃ is -CH₂-Q, wherein Q is alkoxy containing 1 to 3 carbon atoms.

In the compounds of Formula (II), Ar is preferably phenyl unsubstituted or substituted with at least one halo, preferably with at least one fluoro. More preferably Ar in Formula (II) is unsubstituted-phenyl.

In preferred compounds of Formula (III), R₉ is hydrogen or fluoro, R₃ is selected from the group consisting of methoxymethyl, phenyl, N-methoxy-N-methylamino, and N-methoxyamino, and R₁ is methyl.

The most preferred compounds of the present invention include:
(R)-2-acetamido-N-benzyl-3-methoxy-propionamide;
(R)-2-acetamido-N-benzyl-3-ethoxy-propionamide;
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide;
D-α-acetamido-N-(2-fluorobenzyl)-2-furanacetamide;
D-α-acetamido-N-(3-fluorobenzyl)-2-furanacetamide.

It is to be understood that the Markush groups of R₁, R₂, R₃, R and n as described herein can be combined and permutated. The various combinations and permutations not explicitly disclosed herein are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

More preferred is a compound of Formula (I), (II) or/and (III) in the R configuration, preferably substantially enantiopure, wherein the substituent R is benzyl which is unsubstituted with at least one halo group, wherein R₃ is CH₂-Q, wherein Q is alkoxy containing 1-3 carbon atoms and wherein R₁ is methyl. Preferably R is unsubstituted benzyl or benzyl substituted with at least one halo group which is a fluoro group.

Depending upon the substituents, the present compounds may form addition salts as well. All of these forms are contemplated to be within the scope of this invention including mixtures of the stereoisomeric forms.

The manufacture of compounds utilized in the present invention is described in U.S. Patent Nos. 5,378,729 and 5,773,475, and in the international application PCT/EP 2005/010603 the contents of which are incorporated by reference.

The compounds utilized in the present invention are useful as such as depicted in the Formulae (I), (II) or/and (III) or can be employed in the form of salts in view of its basic nature by the presence of the free amino group. Thus, the compounds of Formulae (I), (II) or/and (III) form salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

In a preferred embodiment, the compounds of the present invention are administered in amounts ranging from about 1 mg to about 100 mg per kilogram of body weight per day, more preferably in amounts ranging from about 1 mg to about 10 mg per kilogram of body weight per day. This dosage regimen may be adjusted by the physician to provide the optimum therapeutic response. Patients in need thereof may be treated with doses of the compound of the present invention of at least 50 mg/day, preferably of at least 200 mg/day, more preferably of at least 300 mg/day, still more preferably of at least 400 mg/day and most preferably of at least 600 mg/day. Generally, a patient in need thereof may be treated with doses at a maximum of 6 g/day, more preferably a maximum of 1 g/day, still more a maximum of 800 mg/day, and most preferably a maximum of 600 mg/day. In some cases, however, higher or lower doses may be needed.

In another preferred embodiment, the daily doses are increased until a predetermined daily dose is reached which is maintained during the further treatment.

In yet another preferred embodiment, several divided doses may be administered daily. For example, three doses per day may be administered, preferably two doses per day. It is more preferred to administer a single dose per day.

In yet another preferred embodiment, an amount of the compounds of the present invention may be administered which results in a plasma concentration of 0.1 to 15 µg/ml (trough) and 5 to 18.5 µg/ml (peak), calculated as an average over a plurality of treated subjects, intravenous administration in emergency treatment might result in peak plasmid levels of up to 30 µg/ml.

The compounds of Formulae (I), (II) or/and (III) may be administered in a convenient manner, such as by oral, intravenous (where water soluble), intramuscular, intrathecal, rectal (e.g. suppository, gel, liquid, etc.) or subcutaneous routes. Oral, rectal or/and i.v. administration is preferred. In emergency treatment, i.v. administration is most preferred.

The pharmaceutical composition of the present invention may be prepared for the treatment regimen as described above, in particular for the treatment with doses as described above, to effect plasma concentrations as described above, for administration periods or/and administration routes as specified in the embodiments of the present invention as described above.

The compounds of Formulae (I), (II) or/and (III) may be orally administered, for example, with an inert diluent or with an assimilable edible carrier, or it may be enclosed in hard or soft shell gelatin capsules, or it may be compressed into tablets, or it may be incorporated directly into the food of the diet. For oral therapeutic administration, the active compound of Formulae (I), (II) or/and (III) may be incorporated with excipients and used in the form of ingestible tablets, buccal tablets, troches, capsules, elixirs, suspensions, syrups, wafers, and the like. Such compositions and preparations should contain at least 1 % of active compound of Formulae (I), (II) or/and (III). The percentage of the compositions and preparations may, of course, be varied and may conveniently be between about 5 to about 80 % of the weight of the unit. The amount of active compound of Formulae (I), (II) or/and (III) in such therapeutically useful compositions is such that a suitable dosage will be obtained. Preferred compositions or preparations according to the present invention contains between about 10 mg and 6 g active compound of Formulae (I), (II) or/and (III).

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of preparing sterile powders for the manufacture of sterile injectable solutions, the preferred methods of preparation are vacuum drying, or freeze-drying optionally together with any additional desired ingredient.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is a human.

The term "treat" refers to either relieving the pain associated with a disease or condition, to curing or alleviating the patient's disease or condition.

The compounds of the present invention are administered to a patient suffering from the aforementioned type of disorder in an effective amount. These amounts are equivalent to the therapeutically effective amounts described hereinabove.

The present invention is further illustrated by the following example and figures.

### Figure legends

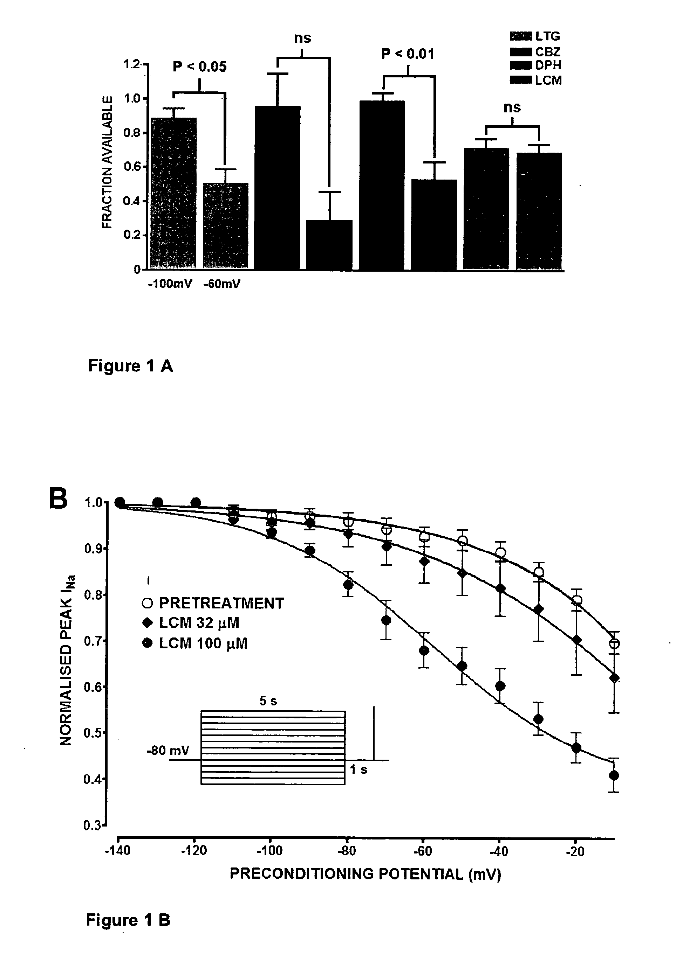

**Figure 1A:** Fraction of channels available after a repolarizing pulse of -100 mV and -60 mV, respectively. LTG: lamotrigine, CBZ: carbamazepine, DPH: phenytoin, LCM: lacosamide.
**Figure 1B:** Voltage dependency of slow inactivation in the presence (LCM) or in the absence (PRETREATMENT) of lacosamide. Cells were held at -80 mV and depolarised for 10 seconds to -10 mV, followed by a recovery interval of 1.5 s prior to a test pulse. The recovery interval of 1.5 s was used to completely allow the recovery of fast inactivation making occupancy of the slow inactivated state the sole determinant of the second test pulse amplitude. The test pulse was used to measure the peak available current.

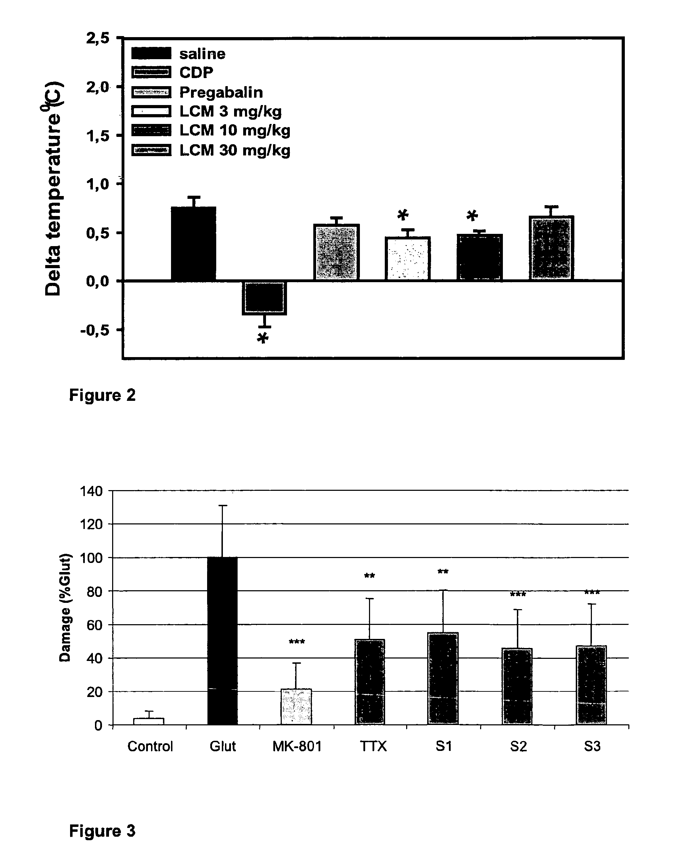

**Figure 2:** Effect of chlordiazepoxide (CDP), pregabalin and lacosamide (LCM, 3 - 30 mg/kg) on SIH. Data represents mean ± SEM of 11-12 mice/treatment group. *: significantly different from saline, P<0.05.

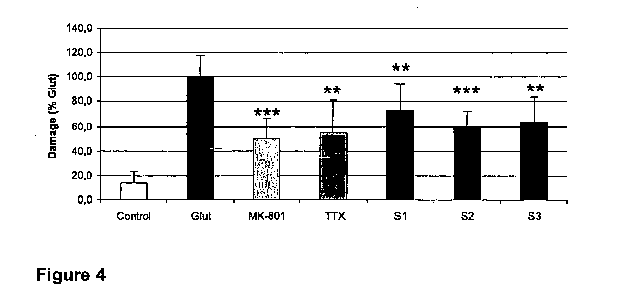

**Figures 3 and 4:** The effects of lacosamide on neuronal damage in CA1-CA3 after glutamate excytotoxicity (Glut). 2h before Glut (15 mM for 24h) cultures were treated with vehicle (Glut), 10µM MK-801, 1µM Tetrodotoxin (TTX), 1µM (S1), 10µM (S2) and 100µM (S3) of lacosamide. Compounds were present from 2h before until 24h after Glut onset. Neuronal damage was assessed by densitometric quantification of propidium-iodide (PI) uptake (**Fig. 3**) or by colorimetric quantification of LDH release (**Fig. 4**) 24h after Glut onset. Glut damage was set to 100%, data are given as % of Glut damage. Data were combined from 2-4 independent experiments Data are given as mean value plus STD (***p<0.001 vs Glut; **p<0.01 vs Glut. Tukey test post hoc after 1-way ANOVA).

### Example 1

Initial studies indicated that lacosamide does not produce changes to the processes of fast inactivation gating of sodium channels in a manner that was consistent with the effects of existing antiepileptic drugs. This was evidenced by a very limited effect of lacosamide upon action potential firing evoked by brief depolarising steps or ramps. The lack of effect of lacosamide upon fast inactivation has been further supported by experiments performed upon Xenopus oocytes expressing only sodium channel alpha subunits. Again, as was observed in neuroblastoma cells, lacosamide did not produce shifts in the fast inactivation voltage curve or retard recovery from steady state fast inactivation. Contrary to the lack of effect of lacosamide, both of these gating processes were considerably changed by carbamazepine, lamotrigine and phenytoin.

In the oocyte expression system lacosamide was however, still able to produce rapid and profoundly voltage dependent inhibition of sodium currents. Sodium channels in mouse neuroblastoma N1E-115 cells undergo the physiological process of slow inactivation in addition to fast inactivation when exposed to periods of prolonged depolarisation. Sodium channels in the fast inactivated conformation can be reactivated by a 500 ms hyperpolarizing pulse. A following test pulse will reveal selectively the amount of slow inactivated sodium channels.

Thus neuroblastoma cells were maintained at a holding potential of -60 mV to induce fast and slow inactivation and depolarised by a 10 ms test pulse to 0 mV to measure the amount of available channels. This protocol was repeated in each cell with a 500 ms hyperpolarizing pulse to -100 mV prior to the depolarising test pulse in order to selectively remove fast inactivation. In all cells tested carbamazepine (CBZ), lamotrigine (LTG), phenytoin (DPH) and lacosamide (LCM, all drugs tested at 100 µM) produced a reduction in current when the holding potential was -60 mV. For CBZ, LTG and DPH application of a 500 ms hyperpolarizing pulse to -100 mV reversed the blocking action on the channel, with the fraction available being 0.94 ± 0.19, 0.88 ± 0.06 and 0.99 ± 0.05 respectively compared to control values (Figure 1 A).

In comparison to the results of the clinically used sodium channel modulating anticonvulsants, the inhibition produced by LCM was not altered by the hyperpolarizing prepulse i.e. by the removal of fast inactivation. This indicates that whereas the classical sodium channel modulating anticonvulsants lamotrigine, carbamazepine and phenytoin selectively act on fast inactivation, lacosamide exerts its effects on sodium currents exclusively by enhancing slow inactivation.

In order to assess concentration-dependent effects of lacosamide on the entry of sodium channels into the slow inactivated state, cells were held at - 80 mV and depolarised for 10 seconds to -10 mV, followed by a recovery interval of 1.5 s prior to a test pulse to measure the peak available current. The recovery interval of 1.5 s was used to completely allow the recovery of fast inactivation making occupancy of the slow inactivated state the sole determinant of the second test pulse amplitude. When neuroblastoma cells were depolarised for 10 seconds the peak available current was reduced to 0.73 ± 0.01 that of the preconditioning test pulse. Lacosamide enhanced the entry of sodium channels into the slow inactivated state in a concentration dependent manner (% of channels in the slow inactivated state: VEH: 27%. LCM 32 µM: 37%, LCM 100µM: 50%, LCM 320 µM: 62%). Lacosamide (100 µM) more than doubled the reduction of sodium channel availability that resulted from entry into the slow inactivated state.

The time constant for the physiological entry into slow inactivation was 12.0 s. In the presence of the drug, channels entered the slow inactivated state faster (i.e. time constant for entry 4.8 s).

As with its more rapid counterpart, the extent that sodium channels undergo the process of slow inactivation at steady state is dependent upon membrane potential. Voltage dependence of slow inactivation in N1E-115 cells was evaluated and revealed physiological slow inactivation at potentials more depolarised than -80 mV. However slow inactivation was only 30 % complete at the maximum conditioning pulse of -10 mV. Lacosamide shifted the slow inactivation voltage curves to more hyperpolarized membrane potentials in a concentration dependent manner. The first significant change in channel availability in the presence of 100 µM LCM was noted at a conditioning potential of -80 mV; more hyperpolarized than the typical resting potential of many neurons. LCM application significantly increased the maximal fraction of current made unavailable by depolarisation across the range of potentials between -80 mV and -10 mV (-10 mV, CONTROL 0.70 ± 0.02, LCM 0.41 ± 0.04). Lacosamide (32 µM and 100µM) produced a shift of the V₅₀ for slow inactivation to more negative potentials (Figure 1 B), i.e. lacosamide shifts the slow inactivation voltage curve for sodium channels to more hyperpolarized potentials. Thus, at the given steady-state potential, the amount of sodium channels which are available for activation is reduced by lacosamide.

The following effects of lacosamide upon sodium channels were identified in rat cortical neurons, NIE-115 cells and Xenopus oocytes:
- Lacosamide did not modulate synaptic transmission under normal physiologic conditions.
- Lacosamide indiscriminately attenuates the rate of synaptic traffic in cultured neurons in a stereoselective and concentration dependent manner.
- The frequency of miniature excitatory post-synaptic potentials is not reduced by lacosamide.
- Lacosamide produced no effect upon resting conductances.
- Lacosamide did not block sustained repetitive firing.
- Lacosamide inhibits slow prolonged bursting in cortical neurons.
- Lacosamide inhibits prolonged sustained repetitive firing on action potentials.
- Lacosamide does not shift steady state fast inactivation voltage curves.
- Lacosamide does not change the rate of sodium channel fast inactivation.
- The rate of recovery of sodium channels from the unavailable slowly inactivated state is not changed.

In summary, these experiments demonstrate that lacosamide selectively enhances slow inactivation of voltage-gated sodium channels while leaving fast inactivation normal. This constitutes a novel mechanism of action which can efficiently normalize excessive sodium channel function as e.g. seen in mutated channels.

In particular, by its effect on slow inactivation of sodium channels, lacosamide is suitable for the prevention, alleviation or/and treatment of diseases associated with hyperexcitability.

### Example 2: Lacosamide in an animal model for stress-induced anxiety

In this study the effects of lacosamide in an animal model for stress-related anxiety were tested. In this animal model, stress is induced by the measurement of rectal temperature.The stress-induced hyperthermia (SIH) test is based upon the principle that mice have a natural hyperthermic response to stress, which reflects the level of stress-induced anxiety. The effects of lacosamide in this model were compared to that of the reference compound chlordiazepoxide (CDP) which is used clinically as first-line anxiolytic treatment. In addition, the novel anticonvulsant drug pregabalin which is also developed for generalized anxiety disorder was used as additional reference compound.

### Materials and methods

Adult male 129SVEV mice (8 weeks old) from Taconic Laboratories (Germantown, NY) were used in this study. The mice were housed in standard polycarbonate cages with filter tops. Four animals were housed per cage and food and water were provided ad libitum for the duration of the study except noted otherwise. Prior to initiation of the study, the animals were examined to assure adequate health and suitability; the animals also received two days of handling prior to test to minimize non-specific stress associated with the test. Mice were maintained on a 12 /12 light/dark cycle with the light on at 6:00 a.m. The room temperature was maintained between 20 and 23°C with a relative humidity maintained between 30% and 70%.

Chlordiazepoxide (CDP 10 mg/kg; batch number 94H1023, Sigma) was dissolved in sterile water. Lacosamide (batch number WE11837 (537.1008,SIFA)) and pregabalin (batch number HS3730) were dissolved in saline. All drugs were administered i.p. 60 min prior to testing.

The test involves two measures of rectal temperature repeated in the same animal with a 10-minute interval. On the day prior to testing, the mice were brought to the experimental room one hour before scheduled lights out and singly housed overnight with food and water ad libitum. On the morning of the experiment, animals were first injected with either saline, CDP (10 mg/kg) or with lacosamide (3, 10 or 30 mg/kg). One hour following injection, each animal was removed from the holding cage and held in a supine position and his rectal temperature was measured by slowly inserting a rectal probe into the animal's rectum at a length of approximately 0.5 cm. The rectal probe is attached to a PhysiTemp digital thermometer (Fisher Scientific) which provides temperature readings at 0.1°C accuracy. The probe remained inside the animal for approximately 5 seconds or until body temperature reached stability. This temperature was recorded as the baseline rectal temperature (T1). The animal was immediately placed back to the holding cage and after a 10-min interval the 2nd rectal temperature (T2) was taken using the same procedure as in measuring T1. Upon completion of the two rectal temperature measures, the animal was returned to the home cage and then later returned to the colony room. Before each insertion, the rectal probe was cleaned with an alcohol pad and lubricated with sterile K-Y jelly. All SIH studies were conducted between 8:00 - 11:00 a.m.

All data was analyzed using one way analysis variance (ANOVA) followed by Fisher PLSD post hoc analysis. Outliers above and below 2 standard deviations from the mean were taken out from the final analysis.

### Results

One way ANOVA found a significant treatment effect. Fisher PLSD post hoc analysis revealed that the reference compound CDP (10 mg/kg) significantly decreased the temperature of the mice compared to the saline-treated mice (Figure 2). Similarly, at doses of 3 and 10 mg/kg, lacosamide significantly attenuated SIH compared to saline (Figure below). No significant effects of either pregabalin or lacosamide (30 mg/kg) were found on SIH.

### Conclusion

Lacosamide is effective in this animal model for stress-induced anxiety. Thus, the compounds of the present invention, in particular lacosamide, are suitable for the prevention, alleviation, or/and treatment of a disease associated with hyperexcitability, such as anxiety or/and stress.

### Example 3

The focus of the present study was the analysis of potential neuroprotective effects of lacosamide in rat hippocampal slice cultures after glutamate (Glut) exposure as a model for a excitotoxic injury. Lacosamide was administered 2h before insult and present until the end of the experiment at 24h after insult. Quantitative analysis of necrotic cell death was performed by propidium iodide (PI) uptake (neuronal cell death in area CA1-CA3) and lactate dehydrogenase (LDH) release (cell death in whole slice). Lacosamide showed a protective effect against necrotic cell death after Glut. Due to the neuroprotective effects found in this study, lacosamide is predicted to be effective for treatment of disorders associated with excitotoxic insults.

### Experimental methods

### Model system: Organotypic hippocampal slice cultures (OHC).

Organotypic slice cultures and among them, hippocampal slice cultures represent in vitro models that keep the different cell types and retain the complex three-dimensional organization of the nervous tissue. Since OHC combine the accessibility/speed of an *in vitro* system and the retained 3-D structure of the respective tissue found *in vivo* they represent an excellent model system for the quick evaluation of the effect of compounds on cell proliferation/differentiation in the CNS. OHC greatly reduce the number of experimental animals and time needed. Furthermore they are perfectly suited for detecting metabolism-related effects, pharmaco-toxical mechanisms and even for assessing effects on overall function.

### Injury Model

7-8-week OHC interphase cultures were prepared according to Stoppini et al. (1991, J Neurosci Methods, 37(2):173-82). OHC (400µM slice thickness) were obtained from young animals (rats; postnatal day 7-9); for the first 2 days the cultures were maintained in medium supplemented with 25% horse serum to facilitate recovery. After that serum-free (Neurobasal) medium was used throughout the experiment. Serum free cultures are better suited for the analysis of exogenously applied factors since unwanted effects of serum components are excluded. On d11 cultures were incubated with 2.5 µM PI for 12h followed by pre-selection to make sure only high quality slices (no PI uptake, no anatomical damage) were used in the experiments. After 12d OHC were treated with lacosamide (1, 10, and 100 µM), a vehicle control (DMSO) and a reference substance 2hrs before the insult. 24h after onset of insult the cultures were analyzed. OHCs were continuously exposed to lacosamide from 2h before until 24h after the neurological insult. Thus lacosamide was present during all stages of the test.

Glutamate induces excitotoxic (necrotic and apoptotic) cell death by acting on all glutamate receptor subtypes: kainate, NMDA and AMPA receptors. OHC were treated with lacosamide or either one of the two neuroprotective reference substances - the NMDA antagonist MK-801 (10 µM) or the Na-channel blocker TTX (1 µM). Two hours afterwards, OHC were subjected to excitotoxic insult by administration of 15mM glutamate for 24h.

### Quantitative assessment of damage

**PI-Uptake (Necrosis) - Assessment of neuronal damage.** 22 h after the respective insult, cultures were stained with propidium iodide (PI) for 2 hrs (PI uptake/incorporation serves as a marker for cell degeneration). Fluorescent images were acquired semi-automatized (Nikon motorized stage; LUCIA software) and analysed by densitometry to quantify necrotic cell death (LUCIA Image analysis software). Damage was analysed only in the CA area (CA1-CA2-CA3) thus representing neuronal damage. PI staining proved to be not suitable for reliable cell death analysis in the AD. In order to combine data from individual experiments the densitometric mean value of the respective insult of an individual experiment was set to 100% damage. All other data are given in % of insult damage.

**LDH Release (Necrosis) - Assessment of general cell death.** Medium samples for this analysis were obtained from the same cultures used for PI-uptake studies. 24h after insult aliquots from cell culture medium (1 well is shared by 2-3 slices) were collected, frozen and kept at 4°C. Subsequent sample analysis of LDH activity was performed using the CytoTox96® (Promega Corp) colorimetric cytotoxicity assay. Measurements were performed on a Magellan Plate reader (492nm vs. 620nm). This assay detects LDH release from all damaged cells thus representing an assessment of general cell death in the slice culture. In order to combine data from individual experiments the mean value of the respective insult of an individual experiment was set to 100% damage. All other data are given in % of insult damage.

**Experimental setup and area of analysis.** On d12 in vitro only high quality slices were selected and treated with lacosamide (1, 10 and 100 µM) 2h before insult onset. lacosamide was present from 2h before until 24h after insult onset. 24h after the insult the assay was terminated and cultures used for analysis: 1) Culture medium samples were obtained for LDH release measurement. 2) PI uptake was analysed in the cultures. Neuronal damage was assessed by densitometric quantification of PI uptake in area CA1 and CA3. In all figures error bars indicate standard deviation. Data were analysed by one-way ANOVA followed by Tukey all pairwise multiple comparison post-hoc test. Statistical significance is defined as * p<0.05, ** p<0.01, or *** p<0.001.

### Results

### Effects of lacosamide after glutamate (Glut) insult

**Neuronal Damage.** As assessed by densitometric PI uptake analysis lacosamide had a statistically significant effect on neuronal damage in all CA subfields (combined CA1, CA2, CA3) 24h after Glut (Fig. 3). This was true for all three concentrations of lacosamide tested (p<0.01 S1 vs. Glut; p<0.001 S2 and S3 vs. Glut). The reference substance MK-801 (10µM) provided significant protection against Glut-induced neuronal damage (p<0.001 for MK-801 vs. Glut). The second reference substance Tetrodotoxin (1µM TTX) showed also a neuroprotective effect after Glut (p<0.01; TTX vs. Glut). The extent of lacosamide mediated neuroprotection was similar to that of TTX but smaller than that of MK-801.

**Overall Damage.** As assessed by colorimetric measurement of LDH release, lacosamide had a statistically significant effect on overall damage 24h after Glut (Fig. 4). This was true for all three concentrations of lacosamide tested (p<0.01 S1 and S3 vs. Glut; p<0.001 S2 vs. Glut). The reference substance MK-801 (10µM) provided significant protection against Glut-induced cellular damage (p<0.001 for MK-801 vs. Glut). The second reference substance Tetrodotoxin (1µM TTX) showed also a protective effect after Glut (p<0.01; TTX vs. Glut). The extent of lacosamide mediated cellular protection was similar to that of TTX and MK-801.

### Discussion and conclusions

This study was designed to investigate the potential protective effects of lacosamide after a neurological insult in-vitro. As endpoints two different readouts were used: Specific neuronal necrotic damage in the CA region was quantified by densitometric PI uptake analysis and general cellular necrotic damage was assessed by measurement of LDH release from the whole slice.

High glutamate levels (Glut) caused massive neuronal necrotic cell death (CA1 more affected than CA3). Administration of the NMDA antagonist MK-801 reduced the neuronal damage by app. 80% and overall damage by app. 50%. This indicates that MK-801 provided protection for neurons but not for glial cells. The Na-channel blocker TTX provided app. 50% of neuronal damage reduction and app. 20-40% of general damage reduction.

Lacosamide showed a significant protective effect on neuronal (CA1 and CA3) and general necrotic damage after Glut. Lacosamide significantly reduced Glut-induced necrosis by app. 40-50% and overall damage by app. 30-40%. According to these data lacosamide seems to have an anti-necrotic effect in a model of excitotoxic insult (Glut).

Thus, the compounds of the present invention, in particular lacosamide, may be suitable for neuroprotective prevention, alleviation or/and treatment in particular in a neurodegenerative disease or/and psychotic disease.

## Claims

1. Use of a compound having the Formula (I) 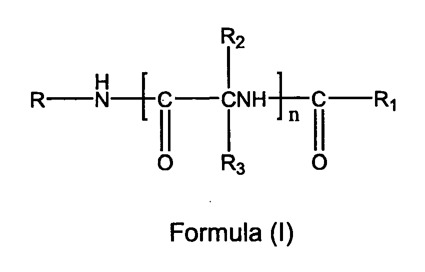 wherein
R is hydrogen, alkyl, alkenyl, alkynyl, aryl, aryl alkyl, heterocyclic, heterocyclic alkyl, alkyl heterocyclic, cycloalkyl or cycloalkyl alkyl, and R is unsubstituted or is substituted with at least one electron withdrawing group or/and at least one electron donating group;
R₁ is hydrogen or alkyl, alkenyl, alkynyl, aryl alkyl, aryl, heterocyclic alkyl, alkyl heterocyclic, heterocyclic, cycloalkyl, cycloalkyl alkyl, each unsubstituted or substituted with at least one electron donating group or/and at least one electron withdrawing group;
R₂ and R₃ are independently hydrogen, alkyl, alkenyl, alkynyl, alkoxy, alkoxyalkyl, aryl alkyl, aryl, halo, heterocyclic, heterocyclic alkyl, alkyl heterocyclic, cycloalkyl, cycloalkyl alkyl, or Z-Y wherein R₂ and R₃ may be unsubstituted or substituted with at least one electron withdrawing group or/and at least one electron donating group; and wherein heterocyclic in R₂ and R₃ is furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl or, when N is present in the heterocyclic, an N-oxide thereof;
Z is O, S, S(O)ₐ, NR₄, NR₆' or PR₄ or a chemical bond;
Y is hydrogen, alkyl, aryl, aryl alkyl, alkenyl, alkynyl, halo, heterocyclic, heterocyclic alkyl, alkyl heterocyclic and Y may be unsubstituted or substituted with at least one electron donating group or/and at least one an electron withdrawing group, wherein heterocyclic has the same meaning as in R₂ or R₃ and, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R_{5,} PR₄SR₇, NR₄PR₅R₆, PR₄NR₅R₇, or N⁺R₅R₆R₇, 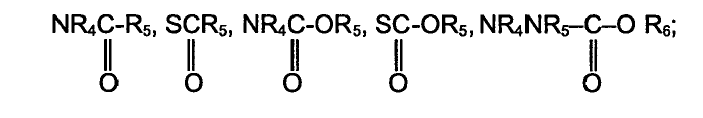
R₆' is hydrogen, alkyl, alkenyl, or alkynyl which may be unsubstituted or substituted with at least one electron withdrawing group or/and at least one electron donating group;
R₄, R₅ and R₆ are independently hydrogen, alkyl, aryl, aryl alkyl, alkenyl, or alkynyl, wherein R₄, R₅ and R₆ may independently be unsubstituted or substituted with at least one electron withdrawing group or/and at least one electron donating group; and
R₇ is R₆ or COOR₈ or COR₈, which R₇ may be unsubstituted or substituted with at least one electron withdrawing group or/and at least one electron donating group;
R₈ is hydrogen or alkyl, or aryl alkyl, and the aryl or alkyl group may be unsubstituted or substituted with at least one electron withdrawing group or/and at least one electron donating group; and
n is 1-4; and
a is 1-3,
or of a pharmaceutically acceptable salt thereof,
for the preparation of a pharmaceutical composition for the prevention, alleviation or/and treatment of a disease associated with hyperexcitability, preferably a disease associated with a hyperexcitable tissue.

2. The use of claim 1, wherein the disease is a disease associated with dysfunction of an ion channel, preferably a voltage-gated ion channel or a ligand-gated ion channel.

3. The use of claim 2, wherein the voltage-gated ion channel is selected from a voltage-gated sodium channel, a voltage-gated calcium channel, a voltage-gated potassium channel, and a voltage-gated chloride channel or/and the ligand-gated ion channel is selected from a nicotinic acetylcholine receptor, a ryanodine receptor, a cyclic nucleotide-gated receptor, an ATP-receptor, a GABA-A receptor, a glutamate-NMDA receptor, a glycine-receptor, a 5-HT3-receptor, and a pH sensitive channel.

4. The use of any of the claims 1 to 3, wherein the disease is a channelopathy.

5. The use of any of the claims 1 to 4, wherein the disease is a muscle disorder.

6. The use of claim 5, wherein the disease is a skeletal muscle disorder or/and a movement disorder, in particular a movement disorder.

7. The use of claim 6, wherein the skeletal muscle or/and movement disorder is selected from myotonias and paralyses, preferably selected from inherited myotonia and periodic paralyses, more preferably selected from paramyotonia congenita, potassium aggravated myotonia, myotonia fluctuans, myotonia permanens, acetazolamide responsive myotonia, hyperkalemic periodic paralysis, normokalemic paralysis, paroxysmal dystonia, Morvan syndrome and Isaak syndrome.

8. The use of claim 6, wherein the skeletal muscle or/and movement disorder is selected from ataxias, in particular selected from episodic ataxia 2, spinocerebellar ataxia 6, and episodic ataxia.

9. The use of claim 6, wherein the skeletal muscle or/and movement disorder is selected from myotonias, in particular selected from Thomsen myotonia, Becker myotonia, myotonia congenita, generalized myotonia, myotonia levior.

10. The use of claim 6, wherein the skeletal muscle or/and movement disorder is a myasthenia selected from myokymias and hypokalemic periodic paralysis 1.

11. The use of claim 5, wherein the muscle disorder is a cardiac arrhythmia selected from long QT syndrome 3, Long QT syndrome 5, Jervell- and Lange-Nielsen syndrome, inducible long QT syndrome, long QT syndrome 1, and long QT syndrome 2.

12. The use of any of the claims 1 to 4 wherein the disease is an epilepsy syndrome selected from generalized epilepsy with febrile seizures plus (GEFS+), severe myoclonic epilepsy in infancy (SMEI), benign familial neonatal infantile seizures (BNIFS), intractable childhood epilepsy with generalized tonic-clonic seizures (ICEGTC), infantile spasms (West syndrome), generalized epilepsy associated with CACNB4 dysfunction, benign familial neonatal convulsions 1, benign familial neonatal convulsions 2, and nocturnal frontal lobe epilepsy.

13. The use of any of the claims 1 to 4, wherein the disease is a pain syndrome selected from erythermalgia and familial rectal pain.

14. The use of any of the claims 1 to 4, wherein the disease is selected from dominant deafness, malignant hyperthermia 5, malignant hyperthermia 1, polycystic kidney disease 1, Dent's disease, Bartter syndrome, central core disorder, and cortical hyperexcitability associated with CACNA1A.

15. The use of any of the claims 1 to 4, wherein the disease is selected from anxiety, stress, posttraumatic stress disorder, and peripheral nerve hyperexcitability syndromes.

16. The use of any of the claims 1 to 4, wherein the disease associated with hyperexcitability is a condition associated with neuronal damage or/and neurodegeneration.

17. The use of claim 16, wherein the disease associated with hyperexcitability is a condition caused by a neurodegenerative disease or/and a psychotic disease preferably selected from Alzheimer's disease, Parkinson's disease, bipolar disorder, and schizophrenia.

18. Use according to any one of the preceding claims, wherein one of R₂ and R₃ is hydrogen.

19. Use according to any one of the preceding claims wherein n is 1.

20. Use according to any one of the preceding claims wherein R is aryl alkyl, especially benzyl, wherein R is unsubstituted or substituted with at least one electron donating group or/and at least one electron withdrawing group.

21. Use according to any one of the preceding claims, wherein R₁ is alkyl which is unsubstituted or substituted with at least one electron donating group or/and at least one electron withdrawing group.

22. Use according to any one of the preceding claims, wherein R₂ and R₃ are independently hydrogen, alkyl, alkoxy or aryl alkyl which is unsubstituted or substituted by at least one electron donating group or/and at least one electron withdrawing group, particularly alkoxyalkyl, heterocyclic, heterocyclic alkyl, or ZY;
wherein Z is O, NR₄ or PR₄; and Y is hydrogen or alkyl; or 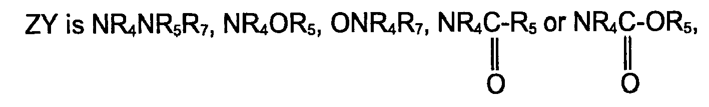 wherein R₄, R₅ and R₇ are as defined in claim 1.

23. Use according to any of the preceding claims, wherein the at least one electron withdrawing or/and at least one electron donating groups is independently selected from halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide.

24. Use according to any one of the preceding claims, wherein at least one electron donating group or/and at least one electron withdrawing group in R₂ or/and R₃ is independently hydroxy or alkoxy.

25. Use according to any one of the claims 1 to 24 wherein the compound has the general Formula (II), 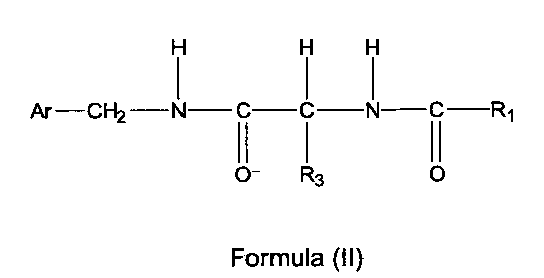 wherein
Ar is aryl, which is unsubstituted or substituted with at least one electron donating group or/and at least one electron withdrawing group;
R₁ is alkyl, and
R₃ is as defined in claim 1, 18, 22, or 24.

26. Use according to claim 25, wherein R₃ is -CH₂-Q, wherein Q is alkoxy.

27. Use according to claim 25 or 26, wherein R₁ is methyl.

28. Use according to any one of the claims 25 to 27, wherein Ar is phenyl unsubstituted or substituted preferably with at least one halo, more preferably at least one fluoro.

29. Use according to claim 28, wherein Ar is unsubstituted phenyl.

30. Use according to claim 28, wherein the compound is
(R)-2-acetamido-N-benzyl-3-methoxy-propionamide;
(R)-2-acetamido-N-benzyl-3-ethoxy-propionamide;
O-methyl-N-acetyl-D-serine-m-fluorobenzylamide; or
O-methyl-N-acetyl-D-serine-p-fluorobenzylamide;

31. Use according to any one of the claims 1 to 30, wherein the compound has the general Formula (III), 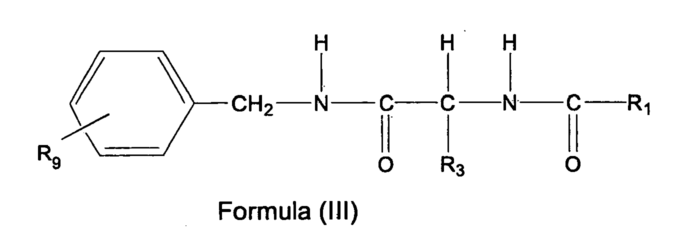 wherein
R₉ is one or more substituents independently selected from the group consisting of hydrogen, halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide;
R₃ is selected from the group consisting of hydrogen, alkyl, alkoxy, alkoxyalkyl, aryl, heterocyclic, heterocyclic alkyl, N-alkoxy-N-alkylamino, N-alkoxyamino, and N-carbalkoxy; and
R₁ is alkyl.

32. Use according to claim 31, wherein R₉ is hydrogen or fluoro, ethoxymethyl and/or R₁ is methyl.

33. Use according to claim 31 or 32, wherein R₃ is selected from alkoxyalkyl, especially methoxymethyl and methoxyethyl, alkyl, especially methyl and ethyl, -NH-O-CH₃, -N(CH₃)-O-CH₃, -NH(C(O)-O-CH₃), aryl, especially phenyl and heterocyclic, especially furyl, pyridinyl, primidinyl, pyrrolyl, oxazolyl and thienyl.

34. Use according to any of claims 31 to 33, wherein the compound is N-acetyl-D-phenylglycinebenzylamide,
D-1,2-(N, O-dimethylhydroxylamino)-2-acetamide acetic acid benzylamide, or
D-1,2-(O-methylhydroxylamino)-2-acetamido acetic acid benzylamide.

35. Use according to any one of the preceding claims wherein the compound is in the R configuration.

36. Use according to claim 35 wherein the compound is substantially enantiopure.

37. Use according to any one of the preceding claims, wherein the pharmaceutical composition is prepared for treatment with doses of the compound at least of 100 mg/day, preferably at least of 200 mg/day, more preferably at least of 300 mg/day, still more preferably at least of 400 mg/day and most preferably of at least 600 mg/day.

38. Use according to any one of the preceding claims, wherein the pharmaceutical composition is prepared for treatment with doses of the compound at a maximum of 6 g/day, more preferably at a maximum of 1 g/day, still more preferably at a maximum of 800 mg/day and most preferably at a maximum of 600 mg/day.

39. Use according to any one of the preceding claims, wherein the pharmaceutical composition is prepared for treatment with doses of at least 400 mg/day and preferably 600 mg/day or 800 mg/day.

40. Use according to any one of the preceding claims, wherein the pharmaceutical composition is prepared for an administration resulting in a plasma concentration of 0.1 to 15 µg/ml (trough) and 5 to 30 µg/ml (peak), calculated as an average over a plurality of treated subjects.

41. Use according to any one of the preceding claims, wherein the pharmaceutical composition is prepared for oral, rectal or i.v. administration, preferably for i.v. administration.

42. Use according to any one of the preceding claims, wherein the pharmaceutical composition comprises
(a) at least one compound of Formulae (I), (II), or/and (III) as defined in any of the claims 1 and 18 to 36, preferably lacosamide, and
(b) at least one further active agent for the prevention, alleviation or/and treatment of a disease associated with hyperexcitability.

43. Use according to claim 42, wherein the compounds (a) and (b) are present in a single dosage form.

44. Use according to claim 42, wherein the compounds (a) and (b) are present in separate dosage forms.

45. Use according to any one of the preceding claims wherein the pharmaceutical composition is prepared for administration in mammals, preferably in humans.

46. Pharmaceutical composition comprising
(a) at least one compound of Formulae (I), (II), or/and (III) as defined in any of the claims 1 and 18 to 36, preferably lacosamide, and
(b) at least one further active agent for the prevention, alleviation, or/and treatment of a disease associated with hyperexcitability.

47. A method for the prevention, alleviation or/and treatment of a disease associated with hyperexcitability, comprising administering to a subject in need thereof at least one compound as defined in any of the claims 1 and 18 to 36, in particular lacosamide.

48. A method for the prevention, alleviation or/and treatment of a disease associated with hyperexcitability, comprising co-administering to a subject in need thereof at least one compound as defined in any of the claims 1 and 18 to 36, in particular lacosamide, and a further active agent for the prevention, alleviation or/and treatment of a disease associated with hyperexcitability.

49. A method according to claim 47 or 48, wherein the administration is orally, by injection (e.g. intravenous or intramuscular) or rectally (e.g. suppository, gel, liquid, etc.), preferably by i.v. injection.
